(19) **Europäisches Patentamt / European Patent Office / Office européen des brevets**

(11) **EP 4 328 931 B1**

(12) **EUROPEAN PATENT SPECIFICATION**

(45) Date of publication and mention
of the grant of the patent:
**01.04.2026 Bulletin 2026/14**

(21) Application number: **23193164.3**

(22) Date of filing: **24.08.2023**

(51) International Patent Classification (IPC):
*G16H 50/30* (2018.01)    *G16H 50/20* (2018.01)
*A61B 5/00* (2006.01)    *G16H 40/67* (2018.01)
*A61B 5/024* (2006.01)    *G16H 40/63* (2018.01)

(52) Cooperative Patent Classification (CPC):
**A61B 5/02405; A61B 5/0004; A61B 5/02438;
A61B 5/4343; A61B 5/6801; A61B 5/7275;
A61B 5/7282; G16H 40/63; G16H 40/67;
G16H 50/20; G16H 50/30**

(54) **DELIVERY DATE PREDICTION**

**VORHERSAGE DES LIEFERDATUMS**

**PRÉDICTION DE DATE DE LIVRAISON**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **26.08.2022 US 202263401406 P
22.08.2023 US 202318453572**

(43) Date of publication of application:
**28.02.2024 Bulletin 2024/09**

(73) Proprietor: **Whoop, Inc.
Boston, MA 02215 (US)**

(72) Inventors:
• **CAPODILUPO, Emily Rachel
Boston, MA 02215 (US)**
• **JASINSKI, Summer Rose
Boston, MA 02215 (US)**
• **HOLMES, Kristen Erika
Boston, MA 02215 (US)**

(74) Representative: **Peterreins Schley
Patent- und Rechtsanwälte PartG mbB
Hermann-Sack-Straße 3
80331 München (DE)**

(56) References cited:
**US-A1- 2022 192 585**

• **ROWAN SHON P. ET AL: "Monitoring One Heart to Help Two: Heart Rate Variability and Resting Heart Rate using Wearable Technology in Active Women Across the Perinatal Period", MEDRXIV, 23 April 2022 (2022-04-23), pages 1 - 21, XP093098711, Retrieved from the Internet <URL:https://www.medrxiv.org/content/10.1101/2022.04.22.22274195v1.full.pdf+html> [retrieved on 20231107], DOI: 10.1101/2022.04.22.22274195**
• **SARHADDI FATEMEH ET AL: "Trends in Heart Rate and Heart Rate Variability During Pregnancy and the 3-Month Postpartum Period: Continuous Monitoring in a Free-living Context", JMIR MHEALTH AND UHEALTH, vol. 10, no. 6, 3 June 2022 (2022-06-03), pages e33458, XP093098438, DOI: 10.2196/33458**

## Description

### RELATED APPLICATIONS

This application claims priority to U.S. Pat. App. No. 63/401,406, filed on August 26, 2022.

### TECHNICAL FIELD

The present disclosure relates to the field of predicting a date of delivery for an infant of a pregnant user, more specifically, to a computer-implemented method for predicting a date of delivery, a system for predicting a date of delivery, a computer program product for predicting a date of delivery and a computer-readable medium configured to store the latter.

### BACKGROUND

The American College of Obstetrics and Gynecology defines preterm birth as delivery occurring between 20 and 37 weeks of gestation. Preterm birth occurs at a rate of 10.2 % in the United States with the majority occurring between 34 and 36 weeks. Infants born prematurely have an increased risk of morbidity and mortality with an estimated economic impact of $25 billion a year in the United States alone.

Ongoing studies are evaluating the use of serum biomarkers, genital tract microbiome, salivary hormone and protein concentrations, cervical texture, and genetic profiling for preterm birth risk assessment. Wireless technology for home monitoring of uterine contractions has also been studied without showing a reduction in preterm birth or improvement in preterm birth outcomes. Unfortunately, these and other methods of detecting preterm labor have met with shortcomings.

At the same time, wearable technology has emerged as a method of tracking and collecting in vivo datasets. In particular, wearable physiological monitors can track heart rate variability (HRV) and resting heart rate (RHR) over extended time periods. While these metrics are widely considered a measure of cardiovascular fitness, wearable technology has other purposes, and the applicant has observed that changes in heart rate and heart rate variability can occur over the course of a pregnancy. There remains a need for techniques to use heart rate data from wearable monitors for more timely detection of possible preterm births.

US 2022/192585 A1 (BILIC ALJOSA ET AL), 23 June 2022, discloses predicting a date of delivery for a pregnant user by using the heart rate. Rowan Shon P. ET AL: "Monitoring One Heart to Help Two: Heart Rate Variability and Resting Heart Rate using Wearable Technology in Active Women Across the Perinatal Period", medRxiv, 23 April 2022, and SARHADDI FATEMEH ET AL: "Trends in Heart Rate and Heart Rate Variability During Pregnancy and the 3-Month Postpartum Period: Continuous Monitoring in a Free-living Context", JMIR MHEALTH AND UHEALTH, vol. 10, no. 6, 3 June 2022, disclose data of heart rate variability during pregnancy.

### SUMMARY

The invention is set out in the appended set of claims

### BRIEF DESCRIPTION OF THE DRAWINGS

Other characteristics will be apparent from the accompanying drawings, which form a part of this disclosure. The drawings are intended to further explain the present disclosure and to enable a person skilled in the art to practice it. However, the drawings are intended as non-limiting examples. Common reference numerals on different figures indicate like or similar features.

Figs. 1a-d are flow charts illustrating a computer-implemented method for predicting a date of delivery.
Fig. 2 is a flow chart illustrating a method for acquiring 2100 heart rate data.
Fig. 3 is a flow chart illustrating a method for acquiring 2100 heart related data in sleep states.
Fig. 4 is a flow chart illustrating a signal processing algorithm for generating a sequence of heart rates for a detected heartbeat.
Fig. 5 shows a heart rate variability calculated on a daily basis for a pregnancy term.
Fig. 6a illustrates a pattern of heart rate variability changes corresponding to preterm birth.
Fig. 6b illustrates a pattern of changes in the heart rate variability corresponding to full term birth.
Fig. 7 illustrates a system for predicting a date of delivery.
Fig. 8 illustrates front and back perspective views of a wearable physiological monitoring device.
Fig. 9 illustrates a wearable physiological monitoring device.
Fig. 10 illustrates placement of a wearable physiological monitoring device on a user's wrist.
Fig. 11 illustrates a side view of a physiological monitoring device including a strap.
Fig. 12 illustrates a side view of a wearable physiological monitoring device in which a modular head portion is removably coupled to a strap.

### DETAILED DESCRIPTION

The embodiments will now be described more fully hereinafter with reference to the accompanying figures, in which preferred embodiments are shown. The foregoing may, however, be embodied in many different forms and should not be construed as limited to the illustrated embodiments set forth herein. ,

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately" or the like, when accompanying a numerical value, are to be construed as indicating a deviation as would be appreciated by one of ordinary skill in the art to operate satisfactorily for an intended purpose. Similarly, words of approximation such as "approximately" or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better describe the embodiments and does not pose a limitation on the scope of the embodiments. No language in the specification should be construed as indicating any unclaimed element as essential to the practice of the embodiments.

In the following description, it is understood that terms such as "first," "second," "top," "bottom," "up," "down," "above," "below," and the like, are words of convenience and are not to be construed as limiting terms unless specifically stated to the contrary.

The term "user" as used herein, refers to any type of animal, human or non-human, whose physiological information may be monitored using an exemplary wearable physiological monitoring system.

The term "continuous," as used herein in connection with heart rate data collection, refers to collection of heart rate data at a sufficient frequency to enable detection of individual heartbeats, and also refers to collection of heart rate data continuously throughout the day and night. In this context, "continuous" or "continuously" will be understood to mean continuously to the extent possible by the monitoring hardware, subject to ordinary data acquisition limitations such as sampling limitations associated with converting physical signals into digital data, and physical limitations associated with physical disruptions during use, e.g., temporary displacement of monitoring hardware due to sudden movements, changes in external lighting, loss of electrical power, physical manipulation or adjustment by a wearer, physical displacement of monitoring hardware due to external forces, and so forth. It will also be noted that heart rate data or a monitored heart rate, in this context, may more generally refer to raw sensor data, heart rate data, signal peak data, heart rate variability data, or any other physiological or digital signal suitable for recovering heart rate data as contemplated herein, and that heart rate data may generally be captured over some historical period that can be subsequently correlated to various metrics such as sleep states, activity recognition, resting heart rate, maximum heart rate, and so forth.

The term "computer-readable medium," as used herein, refers to a non-transitory storage hardware, non-transitory storage device or non-transitory computer system memory that may be accessed by a controller, a microcontroller, a microprocessor, a computational system, or a module of a computational system to encode thereon computer-executable instructions or software programs. The "computer-readable medium" may be accessed by a computational system or a module of a computational system to retrieve and/or execute the computer-executable instructions or software programs encoded on the medium. The non-transitory computer-readable media may include, but are not limited to, one or more types of hardware memory, non-transitory tangible media (for example, one or more magnetic storage disks, one or more optical disks, one or more USB flash drives), computer system memory or random access memory (such as, DRAM, SRAM, EDO RAM) and the like.

The term "distal," as used herein, refers to a portion, end or component of a physiological measurement system that is farthest from a user's body when worn by the user.

The term "proximal," as used herein, refers to a portion, end or component of a physiological measurement system that is closest to a user's body when worn by the user.

The term "equal," as used herein, refers, in a broad lay sense, to exact equality or approximate equality within some tolerance.

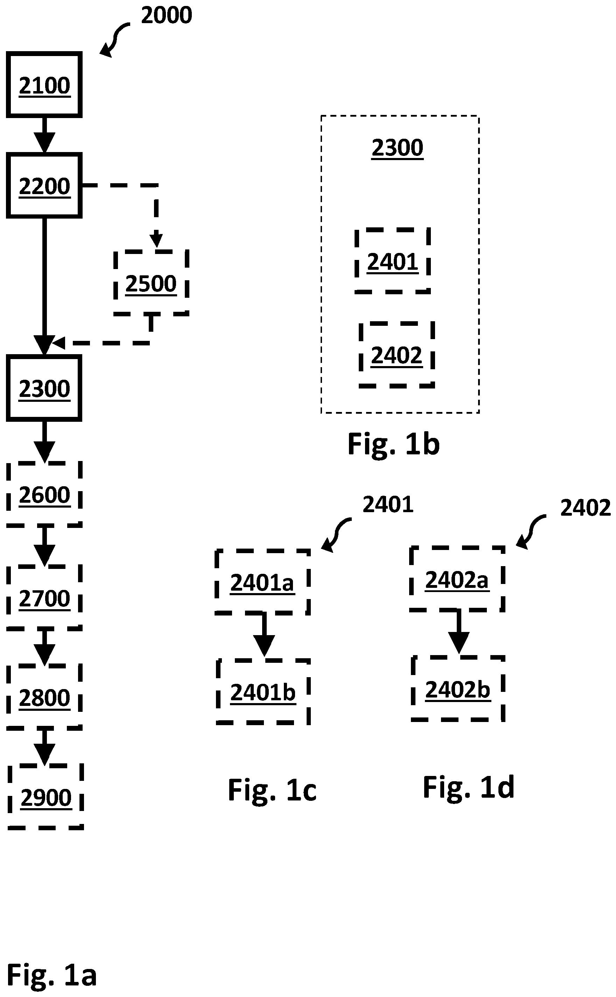

**Figs. 1a-d** are flow charts illustrating a computer-implemented method for predicting a date of delivery. According to the first aspect, the computer-implemented method 2000 for predicting a date of delivery for a pregnant user, comprises acquiring 2100 heart rate data for a pregnant user from wearable sensor system, determining 2200 a heart rate variability and/or a resting heart rate based on the heart rate data, and predicting 2300 the date of delivery using the resting heart rate and/or heart rate variability. The wearable sensor system may be configured to provide two or more different modes for detecting a heart rate of a wearer of the optical sensor system. The modes may include the use of optical detectors (e.g., light detectors), light emitters, motion sensors, a processing module, algorithms, other sensors, a peak detection or other time domain technique, a frequency domain technique, variable optical characteristics, non-optical techniques, and so on. In an example, the method 2000 for predicting a date of delivery may be applied to predict a preterm birth, e.g., by identifying a predicted date of delivery using heart rate data that is different than a predicted date of delivery using a conventional chronology based on the estimated date of pregnancy.

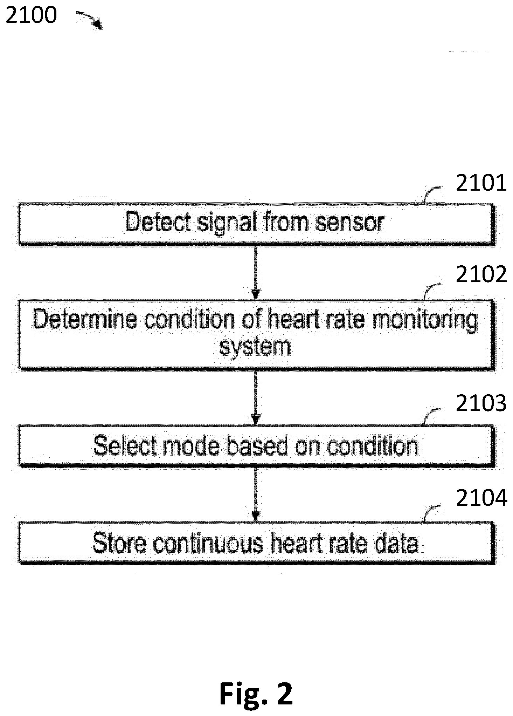

**Fig. 2** is a flow chart of a method for acquiring 2100 heart rate data.

In an example, acquiring 2100 heart rate data may include detecting 2101 a signal from a sensor. The signal may be detected by one or more sensors, which may include any of the sensors described herein. The signal may include, without limitation, one or more signals associated with the heart rate of the user (including ECG, PPG, accelerometer, and audio signals), other physiological signals, an optical signal, signals based on movement, signals based on environmental factors, status signals (e.g., battery life), historical information, and so on.

In an example, acquiring 2100 may comprise determining 2102 a condition of the sensor system, which may be based upon the signal. The condition may include, without limitation, an accuracy of heart rate detection determined using a statistical analysis to provide a confidence level in the accuracy, a power consumption, a battery charge level, a user activity, a location of the sensor (with respect to general space or the user's body), or motion of the sensor, an environmental or contextual condition (e.g., ambient light conditions), a physiological condition, an active condition, an inactive condition, and so on. This may include detecting a change in the condition, responsively selecting a different one of the two or more different modes, and storing additional continuous heart rate data obtained using at least one of the two or more different modes.

In an example, acquiring 2100 may comprise selecting 2103 one of the two or more different modes for detecting the heart rate based on the condition. For example, based on the motion status of the user, the method may automatically and selectively activate one or more light emitters to determine a heart rate of the user. The system may also or instead determine the type of sensor to use at a given time based on the level of motion, skin temperature, heart rate, location on body, and the like. Based on a combination of these factors the system may selectively choose which type of sensor to use in monitoring the heart rate of the user. A processor or the like may be configured to select one of the modes. For example, if the condition is the accuracy of heart rate detection determined using statistical analysis to provide a confidence level in the accuracy, the processor may be configured to select a different one of the modes when the confidence level is below a predetermined threshold.

In an example, acquiring 2100 may comprise storing 2104 continuous heart rate data using one of the two or more different modes. This may include communicating the continuous heart rate data from the sensor system to a remote data repository. This may also or instead include storing the data locally, e.g., on a memory included in the sensor system. The memory may be removable, e.g., via a data card or the like, or the memory may be permanently attached/integral with the sensor system or a component thereof. The stored data (e.g., heart rate data) may be for the user's private use, for example, when in a private setting, the data may be shared when in a shared setting (e.g., on a social networking site or the like), or the data may be uploaded to cloud servers or the like for individual or group use (e.g. as part of an institutional health or wellness program or as part of a remote patient monitoring system). The method 2000 may further include the use of a privacy switch operable by the user to controllably restrict communication of a portion of the data, e.g., to the remote data repository.

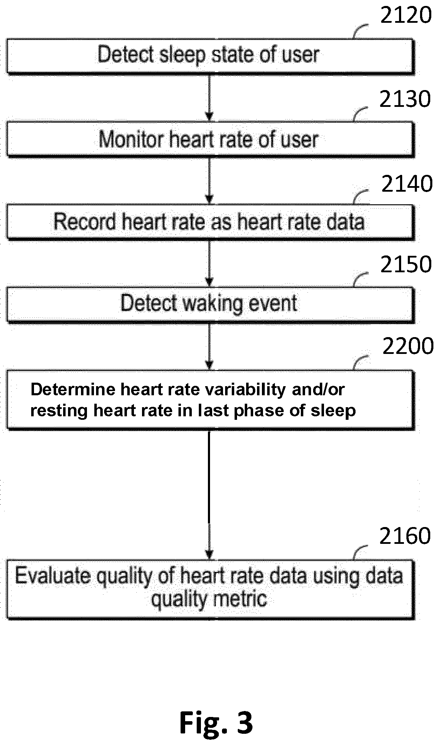

**Fig. 3** is flow chart illustrating a method of acquiring 2100 heart related data in sleep states.

In an embodiment, the acquiring 2100 heart rate data may be performed at night-time when the pregnant user is asleep. For example, the heart rate may be a resting heart rate (RHR). RHR may be calculated based on a specific period of time or sleep stage during the night (similar to the HRV calculations described herein), or an average, or calculated over the non-wake periods of one or more primary sleep episodes, or some other techniques. In an example, to determine 2200 a heart rate variability and/or a resting heart rate based on the heart rate data, the method 2000 may comprise detecting 2120 a sleep state of a user. This may, for example, include any form of continuous or periodic monitoring of sleep states using any of a variety of sensors or algorithms as generally described herein. Sleep states (also be referred to as "sleep phases," "sleep cycles," "sleep stages," or the like) may include rapid eye movement (REM) sleep, non-REM sleep, or any states/stages included therein. The sleep states may include different phases of non-REM sleep, including Stages 1-3. Stage 1 of non-REM sleep generally includes a state where a person's eyes are closed, but the person can be easily awakened; Stage 2 of non-REM sleep generally includes a state where a person is in light sleep, i.e., where the person's heart rate slows and their body temperature drops in preparation for deeper sleep; and Stage 3 of non-REM sleep generally includes a state of deep sleep, where a person is not easily awakened. Stage 3 is often referred to as delta sleep, deep sleep, or slow wave sleep (i.e., from the high amplitude but low frequency brain waves typically found in this stage). Slow wave sleep is thought to be the most restful form of sleep, which relieves subjective feelings of sleepiness and restores the body.

REM sleep on the other hand typically occurs 1-2 hours after falling asleep. REM sleep may include different periods, stages, or phases, all of which may be included within the sleep states that are detected as described herein. During REM sleep, breathing may become more rapid, irregular and shallow, eyes may jerk rapidly (thus the term "Rapid Eye Movement" or "REM"), and limb muscles may be temporarily paralyzed. Brain waves during this stage typically increase to levels experienced when a person is awake. Also, heart rate, cardiac pressure, cardiac output, and arterial pressure may become irregular when the body moves into REM sleep. This is the sleep state in which most dreams occur, and, if awoken during REM sleep, a person can typically remember the dreams. Most people experience three to five intervals of REM sleep each night.

According to the foregoing, sleep of a user may be monitored to detect 2120 various sleep states, transitions, and other sleep-related information. For example, the device may monitor/detect the duration of sleep states, the transitions between sleep states, the number of sleep cycles or particular states, the number of transitions, the number of waking events, the transitions to an awake state, and so forth. Sleep states may be monitored and detected using a variety of strategies and sensor configurations according to the underlying physiological phenomena. For example, body temperature may be usefully correlated to various sleep states and transitions. Similarly, galvanic skin response may be correlated to sweating activity and various sleep states, any of which may also be monitored, e.g., with a galvanic skin response sensor, to determine sleep states. Physical motion can also be easily monitored using accelerometers or the like, which can be used to detect waking or other activity involving physical motion. In another aspect, heart rate activity itself may be used to infer various sleep states and transitions, either alone or in combination with other sensor data. Other sensors may also or instead be used to monitor sleep activity, such as brain wave monitors, pupil monitors, and so forth, depending on the sensor configuration of the monitoring system.

In an example, the method 2000 may comprise monitoring 2130 a heart rate of the user continuously with the (continuous) wearable sensor system. In this context, "continuously" will be understood to mean continuously to the extent possible by the monitoring hardware, e.g., as described herein. More generally, the term "continuously," as used herein in connection with heart rate data, may include the acquisition of heart rate data at a sufficient frequency to enable detection of individual heartbeats, and also refers to the collection of heart rate data over extended periods such as an hour, a day, a week, or more (including acquisition throughout the day and night). Still more generally, with respect to physiological signals that might be monitored by a wearable device, "continuous" or "continuously" will be understood to mean continuously at a rate and duration suitable for the intended time-based processing, and physically at any inter-periodic rate (e.g., multiple times per heartbeat, respiration, and so forth) sufficient for resolving the desired physiological characteristics such as heart rate, heart rate variability, heart rate peak detection, pulse shape, and so forth. At the same time, continuous monitoring is not intended to exclude ordinary data acquisition interruptions such as temporary displacement of monitoring hardware due to sudden movements, changes in external lighting, loss of electrical power, physical manipulation and/or adjustment by a wearer, physical displacement of monitoring hardware due to external forces, and so forth. It will also be noted that heart rate data or a monitored heart rate, in this context, may more generally refer to raw sensor data such as optical intensity signals, or processed data therefrom such as heart rate data, signal peak data, heart rate variability data, or any other physiological or digital signal suitable for recovering heart rate information as contemplated herein. Furthermore, such heart rate data may generally be captured over some historical period that can be subsequently correlated to various other data or metrics related to, e.g., sleep states, recognized exercise activities, resting heart rate, maximum heart rate, and so forth.

In an example, as mentioned above method 2000 may comprise recording 2140 the heart rate as heart rate data. This may comprise storing the heart rate data in any raw or processed form in the sensor system, or transmitting the data to a local or remote location for storage. In one aspect, the data may be stored as peak-to-peak data or in some other semi-processed form without calculating heart rate variability. This may be useful as a technique for conserving processing resources in a variety of contexts, for example where only the heart rate variability at a particular time is of interest. Data may be logged in some unprocessed or semi-processed form, and then the heart rate variability at a particular point in time can be calculated once the relevant point in time has been identified.

In an example, the method 2000 may comprise detecting 2150 a waking event at a transition from the sleep state of the user to an awake state. It should be appreciated that the waking event may be a result of a natural termination of sleep, e.g., after a full night's rest, or in response to an external stimulus that causes awakening prior to completion of a natural sleep cycle. Regardless of the precipitating event(s), the waking event may be detected via the various physiological changes described above, or using any other suitable techniques. While the emphasis herein is on a wearable, continuous monitoring device, it will be understood that the device may also receive inputs from an external device such as a camera (for motion detection), an infrared camera (for body temperature detection), a smart phone or other device with an external audio system (that can detect sounds associated with stirring), and the like that can be used to aid in accurately assessing various sleep states and transitions.

Thus, the wearable sensor system may generally detect a waking event using one or more sensors including, for example, one or more of an accelerometer, a galvanic skin response sensor, a light sensor, and so forth. For example, in one aspect, the waking event may be detected using a combination of motion data and heart rate data.

In an example, determining 2200 the heart rate variability and/or the resting heart rate of the user may be performed at a moment in a last phase of sleep preceding the waking event based upon the heart rate data, or as an average of measurements over a number of sleep cycles, or based on a quality metric for a number of different measurements.

It will be appreciated that the last phase of sleep preceding a natural waking event may be slow wave sleep. However, where a sleeper is awakened prematurely, this may instead include, e.g., a last recorded episode of REM sleep, slow wave sleep, or some other phase of sleep preceding the waking event. This moment - the end of the last phase of sleep before ordinary waking, or the last cycle of the desired phase during which a measurement could be captured - is the point at which heart rate variability data provides the most accurate and consistent indicator of physical recovery. Thus, with the appropriate point of time identified, the historical heart rate data (in whatever form) may be used with the techniques described above to determine 2200 the corresponding heart rate variability and/or resting heart rate. It will be further noted that the time period for this calculation may be selected with varying degrees of granularity depending on the ability to accurately detect the last phase of sleep and an end of the last phase of sleep. Thus, for example, the time may be a predetermined amount of time before waking, or at the end of the last complete cycle of slow wave sleep, or some predetermined amount of time before the end of the last complete cycle of slow wave sleep, or some other predetermined point in the sleep cycles for a user at which consistent measurements can be captured in a manner that supports day-to-day comparisons, analysis, and the like. In another aspect, an average heart rate variability, resting heart rate, or similar metric may be determined for any number of discrete measurements within a window around the time of interest.

In an example, determining 2200 the heart rate variability and/or the resting heart rate may comprise detecting a slow wave sleep period in order to get representative and comparable data. In an example, determining 2200 the heart rate variability and/or the resting heart rate may comprise evaluating 2160 a quality of heart rate data using a data quality metric for a slow wave sleep period, e.g., the slow wave sleep period occurring most recently before the waking event. As noted above, the quality of heart rate measurements may vary over time for a variety of reasons. Thus, the quality of heart rate data may be evaluated prior to selecting a particular moment or window of heart rate data for calculating heart rate variability or calculating resting heart rate. For example, the method 2000 may include calculating the heart rate variability or calculating the resting heart rate for a window of predetermined duration within the slow wave sleep period having the highest quality of heart rate data according to the data quality metric.

In an example, determining 2200 the heart rate variability and/or the resting heart rate of the user may be performed over the course of the night. In an example, determining 2200 may comprise determining of multiple values of the heart rate variability and/or the resting heart rate over the course of the night, which may each be formed of a weighted average of values over different sleep phases, and or may be combined in a weighted or unweighted (e.g., averaged) manner into single measurements of resting heart rate and heart rate variability. This may advantageously make the calculating/determining less sensitive to specific time periods of each measurement, especially when one or more of the measurements occur around a time of waking.

The interval between two heartbeats is usually defined as the time between the onset of two contractions of the heart chambers. This onset of ventricular contraction appears on an electrocardiogram (ECG) as a so-called R-wave. The interval between two R-waves is therefore called the R-R interval. Heart rate variability (HRV) is traditionally measured using an ECG machine that obtains a time series of R-R intervals. In an example, the wearable sensor system may utilize photoplethysmography (PPG), and therefore does not obtain the electric signature from the heart beats; instead, the peaks in the obtained signal correspond to arterial blood volume. At rest, these peaks are directly correlated with cardiac cycles, which enables the calculation of HRV via analyzing peak-to-peak intervals (the PPG analog of RR intervals).

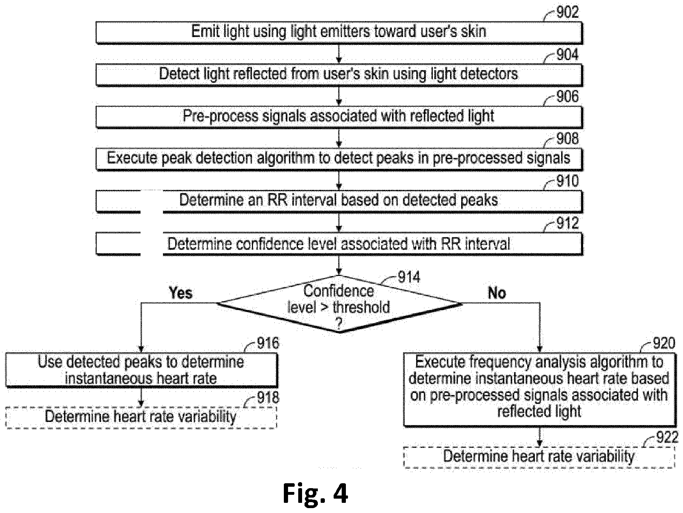

**Fig. 4** illustrates a signal processing algorithm for generating a sequence of heart rates for detected heartbeats. The algorithm may be embodied in computer executable instructions stored on one or more non-transitory computer-readable media. In step 902, light emitters of the wearable sensor system emit light toward a user's skin. In step 904, light reflected from the user's skin is detected at the light detectors in the system. In step 906, signals or data associated with the reflected light are pre-processed using any suitable technique to facilitate detection of heartbeats. In step 908, one or more computer-executable instructions associated with a peak detection algorithm may be performed to process data corresponding to the reflected light to detect a plurality of peaks associated with a plurality of beats of the user's heart. In step 910, an RR interval may be determined based on the plurality of peaks detected by the peak detection algorithm. In step 912, a confidence level associated with the RR interval may be determined.

Based on a confidence level associated with the RR interval estimate, either the peak detection algorithm or a frequency analysis algorithm may be selected to process data corresponding to the reflected light to determine the sequence of instantaneous heart rates of the user. The frequency analysis algorithm may process the data corresponding to the reflected light based on the motion of the user detected using, for example, an accelerometer. In an example, the peak detection algorithm or the frequency analysis algorithm may be selected regardless of a motion status of the user. It is advantageous to use the confidence level when deciding whether to switch to frequency-based methods because certain frequency-based approaches are unable to obtain accurate RR intervals for heart rate variability analysis under certain conditions. Therefore, an implementation maintains the ability to obtain the RR intervals for as long as possible, even in the case of motion, thereby maximizing the information that can be extracted.

For example, in step 914, it may be determined whether the confidence level associated with the RR interval is above (or equal to or above) a threshold. In certain embodiments, the threshold may be predefined, for example, about 50%-90% in some embodiments and about 80% in one non-limiting embodiment. In other embodiments, the threshold may be adaptive, i.e., the threshold may be dynamically and automatically determined based on previous confidence levels. For example, if one or more previous confidence levels were high (i.e., above a certain level), the method may determine that a present confidence level that is relatively low compared to the previous levels is indicative of a less reliable signal. In this case, the threshold may be dynamically adjusted to be higher so that a frequency-based analysis method may be selected to process the less reliable signal.

If the confidence level is above (or equal to or above) the threshold, in step 916, the plurality of peaks may be used to determine an instantaneous heart rate of the user. On the other hand, in step 920, based on a determination that the confidence level associated with the RR interval is equal to or below the predetermined threshold, one or more computer-executable instructions may be executed associated with the frequency analysis algorithm to determine an instantaneous heart rate of the user based on a frequency domain analysis of the PPG signals associated with reflected light. The confidence threshold may be dynamically set based on previous confidence levels.

In some embodiments, in steps 918 or 922, a heart rate variability of the user based on the sequence of the instantaneous heart rates/beats may be determined.

The method may include rendering a user interface for displaying the sequence of the instantaneous heart rates of the user, the RR intervals and/or the heart rate variability.

In one aspect, the method may include switching between two or more different analytical techniques for determining a heart rate such as a statistical technique for detecting a heart rate and a frequency domain technique for detecting a heart rate. These two different modes have different advantages in terms of accuracy, processing efficiency, and information content, and as such may be useful at different times and under different conditions. Rather than selecting one such mode or technique as an attempted optimization, the method may usefully switch back and forth between these differing techniques, or other analytical techniques, using a predetermined criterion. For example, where statistical techniques are used, a confidence level may be determined and used as a threshold for switching to an alternative technique such as a frequency domain technique. The threshold may also or instead depend on historical, subjective, and/or adapted data for a particular user. Suitable statistical techniques for, e.g., probabilistic peak detection, are described by way of non-limiting example, in U.S. Pat. No. 10,264 982. A selection of a threshold for transitioning between techniques may depend on data for a particular user including without limitation subjective information about how a heart rate for a particular user responds to stress, exercise, and so forth. Similarly, the threshold may adapt to changes in fitness of a user, context provided from other sensors of the wearable system (amount of motion, temperature, galvanic skin response, and so forth), signal noise, and so forth. Other techniques for determining the heart rate may also or instead be used, such as simple time domain peak detection.

An exemplary statistical technique employs probabilistic peak detection. In this technique, a discrete probabilistic step may be set, and a likelihood function may be established as a mixture of a Gaussian random variable and a uniform. The heart of the likelihood function encodes the assumption that with a first probability (p) the peak detection algorithm has produced a reasonable initial estimate, but with a second probability (1-p) it has not. In a subsequent step, Bayes' rule is applied to determine the posterior density on the parameter space, of which the maximum is taken (that is, the argument (parameter) that maximizes the posterior distribution). This value is the estimate for the heart rate. In a subsequent step, the previous two steps are reapplied for the rest of the sample. There is some variance in the signal due to process noise, which is dependent on the length of the interval. This process noise becomes the variance in the Gaussians used for the likelihood function. Then, the estimate is obtained as the maximum a posteriori on the new posterior distribution. A confidence value is recorded for the estimate which, for some precision measurement, the posterior value is summed at points in the parameter space centered at our estimate +/- the precision.

The beats per minute (BPM) parameter space, $\theta$, may range between about 20 and about 240, corresponding to the empirical bounds on human heart rates. In an exemplary method, a probability distribution is calculated over this parameter space, at each step declaring the mode of the distribution to be the heart rate estimate. A discrete uniform prior may be set: $\pi_1 \sim$ DiscUnif($\theta$). The un-normalized, univariate likelihood is defined by a mixture of a Gaussian function and a uniform: $l_1 \sim$ IG + (1 - I)U, G ~N($\gamma_1\sigma^2$), $I\sim$ Ber(p), where $U\sim$ DiscUnif($\theta$) and where $\sigma$ and $p$ are predetermined constants. Bayes' rule is applied to determine the posterior density on $\theta$, for example, by component-wise multiplying the prior density vector $(\pi_1(\theta))_{\theta\in\theta}$ with the likelihood vector $(l_1(\theta))_{\theta\in\theta}$ to obtain the posterior distribution $\eta_1$. Then, the following is set: $\beta_1 = argmax_{\theta\in\theta}\eta_1(\theta)$. For $k \geq 2$, the variance in signal S(t) due to process noise is determined. Then, the following variable is set to imbue temporally long RR intervals with more process/interpeak noise and set to post-normalization convolution:

$$\pi_k = \eta_{k-1} {}^*f_{N(o,\lambda_k^2)|\theta} \text{ where } f \text{ is a density function}$$

of the following: $Z \sim N(0, \lambda_k^2)$. Then, the following expressions are calculated: $I_k \sim pG_k + (1 - p)U$, $G_k \sim N(\lambda k, \sigma^2)$. The expression is then normalized and recorded: $\beta_k = argmax_{\theta \in \theta} \eta_k(\theta)$. Finally, the confidence level of the above expression for a particular precision threshold is determined: $C_k = \Sigma_{\theta \in [\beta_k - e_1, \beta_k + e] \cap \theta} \eta_k$. An exemplary frequency analysis algorithm used in an implementation isolates the highest frequency components of the optical data, checks for harmonics common in both the accelerometer data and the optical data, and performs filtering of the optical data. The algorithm takes as input raw analog signals from the accelerometer (3-axis) and pulse sensors, and outputs heart rate values or beats per minute (BPM) for a given period of time related to the window of the spectrogram.

The isolation of the highest frequency components is performed in a plurality of stages, gradually winnowing the window-sizes of consideration, thereby narrowing the range of errors. In one implementation, a spectrogram of 2^15 samples with overlap 2^13 samples of the optical data is generated. The spectrogram is restricted to frequencies in which heart rate can lie. These restriction boundaries may be updated when smaller window sizes are considered. The frequency estimate is extracted from the spectrogram by identifying the most prominent frequency component of the spectrogram for the optical data. The frequency may be extracted using the following exemplary steps. The most prominent frequency of the spectrogram is identified in the signal. It is determined if the frequency estimate is a harmonic of the true frequency. The frequency estimate is replaced with the true frequency if the estimate is a harmonic of the true frequency. It is determined if the current frequency estimate is a harmonic of motion sensor data. The frequency estimate is replaced with a previous temporal estimate if it is a harmonic of the motion sensor data. The upper and lower bounds on the frequency obtained are saved. A constant value may be added or subtracted in some cases. In subsequent steps, the constant added or subtracted may be reduced to provide narrower searches. A number of the previous steps are repeated one or more times, e.g., three times, except taking 2^{15-i} samples for the window size and 2^{13-i} for the overlap in the spectrogram where i is the current number of iteration. The final output is the average of the final symmetric endpoints of the frequency estimation.

In an embodiment, the heart rate variability is a root-mean-square of time differences between two or more heartbeats in a first time period. In an example, a time difference may correspond to one R-R interval. The first time period may correspond to a window of predetermined duration within the slow wave sleep period having the highest quality of heart rate data according to a data quality metric or any other time windows during the user's night at or near a point of temporal interest. The first time period may comprise 70 minutes to 90 minutes, several hours, or several minutes such as 5 minutes to 10 minutes, 10 minutes to 20 minutes, or 20 minutes to 30 minutes.

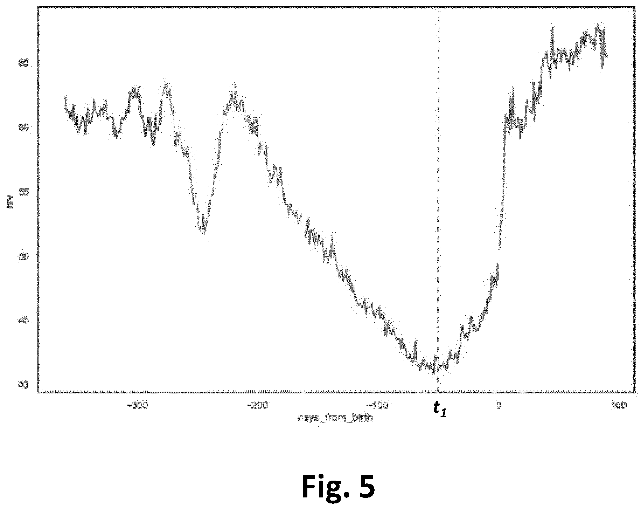

**Fig. 5** shows a heart rate variability calculated on a daily basis for a pregnancy term. More specifically, Fig. 5 illustrates an exemplary pattern of changes in heart rate variability, in daily resolution, spanning a period before, during, and after pregnancy, with an inflection point $t_1$ labeled, and with the birth occurring at t=0.

Heart rate variability, as a metric, characterizes how much the heart rate tends to change over time. However, the heart rate variability may be recorded as a series of measurements/calculations that can, itself, change over time, and these timewise changes in heart rate variability may be used to draw inferences about the physiological state of an individual. In order to advantageously reduce variability of this measurement in response to short term physiological changes, multiple values of a heart rate variability are obtained and combined into a mean or median, which is used as a metric that is representative of the heart rate variability over some desired window or predetermined second time period. In an example, the duration of one night may correspond to the second time period and a plurality of values of a heart rate variability may be determined per night for a plurality of first time periods during the same night, and an average value of heart rate variability representative of heart rate variability for the night may be determined.

In another example, the predetermined second time period may correspond to 7 days. In order to obtain a measure of heart rate variability for such a time period, one or more values of a heart rate variability may be determined per night, such as from several separate and/or overlapping time periods, e.g., from one or more time periods having the highest quality of heart rate data (e.g. in accordance with the quality metric), during one or more specific sleep stages, during one or more specific time periods within sleep stages (e.g., best quality within a sleep stage, a two minute window ending one minute before the end of a sleep state, etc.), and so forth. In one aspect, the heart rate variability may be measured over a window during one or more deep sleep stages, and may be more specifically measured over a predetermined window of time before the end of each deep sleep stage, or before the last deep sleep stage before waking.

In another example, a weighted average value of a heart rate variability and/or a resting heart rate is determined based on multiple values of a heart rate variability and/or a resting heart rate during sleep. In an example, each value of the multiple values of a heart rate variability and/or a resting heart rate may be determined during a first time period of a plurality of first time periods during sleep. A variety of techniques may be used to weight these individual measurements, and the weighting may favor (e.g., weight more heavily) periods of slow wave sleep, specific intervals within periods of slow wave sleep (e.g., the last minute or other window at or near the end of a slow wave sleep cycle), periods of slow wave sleep having the highest quality, sleep periods closer to the end of a sleep episode (e.g., in the morning, or when a user is awakened for the day), or some combination of these or any other intervals during a sleep episode where heart rate variability can more consistently be measured from day to day. For example, in one aspect, heart rate variability may be calculated throughout a sleep episode, with measurements obtained during periods of slow wave sleep weighted more heavily than other sleep stages, and with measurements obtained later in the sleep episode (e.g., as the end of the episode approaches) weighted more heavily than the beginning. The beginning-to-end weighting may be, e.g., a linearly increasing weighting, a sigmoidally increasing weighting, or any other generally monotonically increasing weighting, and this increasing weighting may be applied to all measurements, or only measurements obtained during slow wave sleep.

In an example, the first time period for determining a value of the multiple values of the heart rate variability and/or the resting heart rate during sleep may be between 4 to 6 minutes, more preferably about 5 minutes. In an example, a subsequent first time period of the plurality of first time periods during sleep may begin a predetermined time period after the beginning of the previous first time period of the plurality of first time periods during sleep. That is, the time periods may be staggered, overlapping intervals each with a predetermined duration and each having a starting time offset from the prior time period by a predetermined offset. In another aspect, the time periods may be non-overlapping time periods. For example, the time periods may each have a predetermined duration beginning at a predetermined offset after an end of the prior time period. In an example, the predetermined time period after the beginning of the previous first time period may be between 0 to 60 seconds, preferably between 20 to 40 seconds, more preferably about 30 seconds. In this manner, heart rate variability measurements may be obtained throughout a sleep episode, e.g., for five-minute overlapping intervals with windows having starting times separated by about thirty seconds. In general, overlapping windows may provide greater accuracy and resilience to noise, interference, data interruptions and the like. On the other hand, non-overlapping windows may usefully decrease the amount of data that is gathered over a period of sleep.

In general, any such technique may suitably by selected, preferably in a manner that provides consistent measurements over time, e.g., by choosing one or more specific windows and offsets within the user's sleep each day. In this manner, a more consistent and accurate baseline can advantageously be established for detecting changes in HRV indicative of gestation period as described herein. In one aspect, a representative mean or median of a heart rate variability (measured over any of the intervals described above) for an interval such as 7 days, may be determined. As a significant advantage, smoothing the heart rate variability with a 7-day moving average (or median), or other multi-day moving average, may help to further improve the signal to noise ratio due to daily fluctuations related to things like sleep, activity level, stress, hydration, nutrition, and so forth.

In the example illustration of Fig. 5, the heart rate variability was calculated based on a sequence of measurements during sleep, more specifically as a root mean square of the heart rate variability for five-minute epochs, separated by thirty second intervals. The resulting heart rate variability measurements may then be filtered to remove epochs that fail to meet certain quality metrics (e.g., detectable R-R intervals), and weighted throughout the sleep. For example, this may include weighting according to a probability that each epoch is during slow wave sleep (with greater weight for more likely slow wave sleep), and weighting according to time of night (e.g., by scaling with a sigmoid function or the like). The weighting may generally be normalized or scaled so that the sum of weights or scales for each adjustment sum to one over the set of accumulated epochs. While a specific calculation was used to determine the daily heart rate variability illustrated in Fig. 5, it will be understood that other intervals, weightings, scalings, and the like may also or instead be used, provided they produce a suitably stable and consistent measurement of daily heart rate variability to detect the longer term (e.g., greater than one day) changes described herein.

While a root mean square calculation is described, it will be understood that numerous other techniques may also or instead be used to calculate a heart rate variability for use as further described herein, such as any of the following techniques. In one aspect, time domain methods may be used for calculating heart rate variability, such as Standard Deviation of all NN intervals ("SDNN"), where NN intervals are all the intervals between adjacent QRS complexes resulting from sinus node depolarizations; the square root of the mean squared differences of successive NN intervals ("RMSSD"); the number of pairs of adjacent NN intervals differing by more than 50 ms ("NN50"); the proportion derived by dividing NN50 by the total number of NN intervals ("pNN50"), and so forth. In another aspect, frequency domain methods may be used to decompose the heart rate signal into its frequency components, e.g., by using high frequency power (HF) as a measure of parasympathetic (vagal) activity, or a ratio of low frequency to high frequency power (LF/HR Ratio) as an index of the balance between sympathetic and parasympathetic activity. Other non-linear methods may also or instead be used, such as Poincaré plots, entropy, and detrended fluctuation analysis. More generally, any metric suitable for measuring variations in heart rate activity, and/or detecting changes in the balance between sympathetic and parasympathetic activity, may be used as a measure of heart rate variability for the further purposes discussed herein.

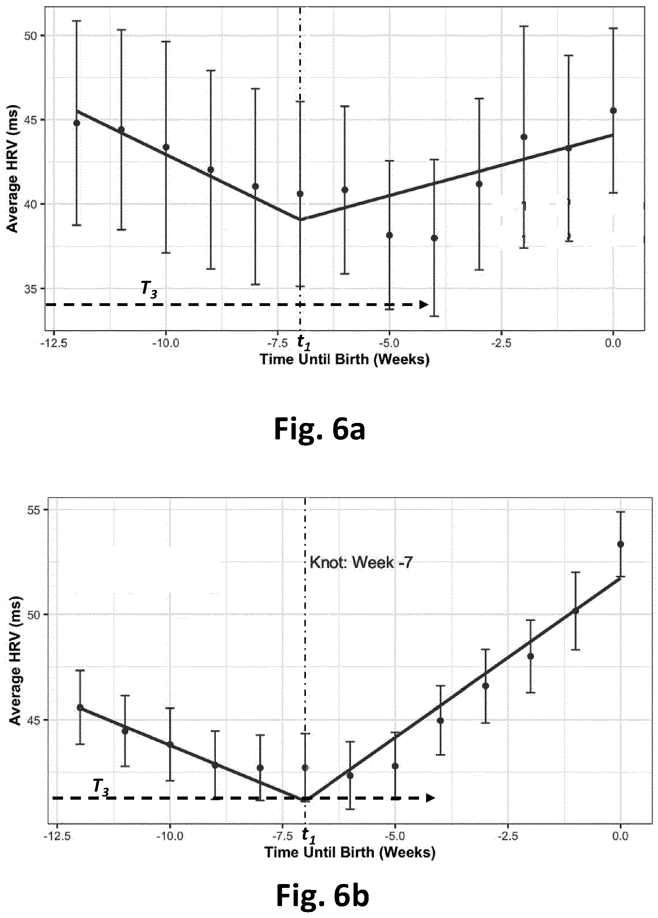

**Fig. 6a** illustrates a pattern of heart rate variability changes corresponding to preterm birth in weekly resolution, based on a weekly calculation of a seven-day moving median of the daily heart rate variability. While a daily heart rate variability calculation may be used to identify an inflection point indicating an approaching birth date as described herein, and such embodiments are intended to fall within the scope of this disclosure, a smoothed metric such as a seven day moving median, a seven day moving average, or other timewise smoothed measure may advantageously be employed to avoid a false positive detections of an inflection based on unrelated daily fluctuations in heart rate variability.

**Fig. 6b** illustrates a pattern of changes in the heart rate variability corresponding to full term birth in weekly resolution, based on a weekly calculation of a seven-day moving median of the daily heart rate variability.

Returning to Fig. 1a, in an embodiment, the predicting 2300 comprises determining 2401 an inflection point based on a pattern of change in the heart rate variability over a third time period $T_3$, (in Figs. 6a & 6b) wherein the third time period $T_3$ may result from a sequence of second time periods. The third time period $T_3$ indicates the gestational age at the time point of predicting 2300 the date of delivery. In an example, a gestational age may be specified in terms of weeks, such that the second time period is one week (7 days) and the third time period $T_3$ is several weeks. In an example, the third time period $T_3$ may comprise 33-42 weeks, 33-40 weeks, 36-42 weeks, 36-40 weeks, more than 36, or less than 36 weeks. A pattern of change comprises a trend change in the time-dependent heart rate variability function. The rest of this paragraph is not part of the invention. The inflection point is defined in the appended set of claims. For example, at the inflection point $t_1$, the function of the heart rate variability may take a value that is smaller than the majority of the values of the heart rate variability in the previous time interval, or smaller than a majority of values in a window around the inflection point $t_1$. In an example, at or after the inflection point $t_1$, the function of the heart rate variability may take a value that is greater than the majority of the values in the previous time interval. In another aspect, the inflection point may be identified where the function of heart rate variability meets or exceeds a predetermined threshold value, which may be determined, e.g., based on a window of prior values, a window of surrounding values, or some other individual, aggregated, or other statistical measurement for an individual.

The pattern of change is defined by the heart rate variability decreasing over time until the inflection point $t_1$ and increasing after the inflection point $t_1$. **Fig. 6a** and **Fig. 6b** both showing a heart rate variability decreasing until the inflection point and increasing after the inflection point, for example, indicating a time of 7 weeks until the date of delivery (date of birth). The inflection point $t_1$ is a value of the abscissa at which the corresponding ordinate value, e.g., the corresponding heart rate variability, has a smallest value. After the inflection point, the value of the heart rate variability increases compared to the value corresponding to the inflection point $t_1$. In other words, in this embodiment, at the inflection point $t_1$ the heart rate variability turns from decreasing to increasing.

**Fig. 1c** illustrates a method of determining 2401 the inflection point $t_1$ using a regression function.

In an embodiment, determining 2401 the inflection point $t_1$ may comprise estimating 2401a a regression function using the heart rate variability, and determining 2401b the inflection point $t_1$ corresponding to a minimum of the regression function. A regression function may be a linear regression function. In one example, the values of the heart rate variability timewise before the inflection point $t_1$ may be part of a first regression function indicating decreasing values of the heart rate variability, and the values of the heart rate variability timewise after the inflection point $t_1$ may be part of second regression function indicating increasing values of the heart rate variability. For example, two (or more) regression functions may be combined to form a composite regression function, which may also or instead be a continuous regression function, such as a continuous regression function that is non-differentiable at the inflection point $t_1$, and/or a polynomial regression function such as a quadratic regression function or a multiple linear regression function.

Furthermore, an abscissa value, e.g., the inflection point $t_1$, may be determined at which the regression function reaches a minimum. A minimum may comprise a minimum over a certain interval. A minimum may be a global or a local minimum. In a preferred embodiment, the minimum is a global or absolute minimum of the regression function within the third time period $T_3$. For example, determining 2401 an inflection point $t_1$ may be performed sometime after inflection point $t_1$ is actually reached to verify that an absolute minimum occurred at the inflection point $t_1$. For example, the determining 2401 may be performed 3 weeks after the actual occurrence of the inflection point $t_1$ to confirm that the value of the heart rate variability has reached a global minimum at the inflection point $t_1$ and the rising trend of the heart rate variability continues after the inflection point $t_1$. Or, in other words, at any point in time (within the third time period $T_3$) the last one or more (for example 3 to 6) weeks may be examined for a global minimum of the heart rate variability or the inflection point in the function of the heart rate variability.

In an embodiment, the resting heart rate may be a function of time and may comprise multiple values of a resting heart rate, wherein each value of the multiple values of the resting heart rate represents a mean or median of the resting heart rate of a predetermined second time period. In an example, the duration of one night may correspond to the second time period and a plurality of values of a resting heart rate may be determined per night for a plurality of first time periods during the same night, and an average value of the resting heart rate representative of a resting heart rate for the night may be determined. In another example, the predetermined second time period may correspond to 7 days. In an example, one or more values of the resting heart rate may be determined per night, either from individual different first time periods or from a first time period of the data having the highest quality (e.g., in accordance with the quality metric), and then a representative mean or median of the resting heart rate for 7 days may be determined. In an example, smoothing the resting heart rate with a 7-day moving median or mean may help to improve the signal to noise ratio due to daily fluctuations related to things like sleep, activity level, stress, and hydration/nutrition. In other examples, the second time period may correspond to any suitable time duration, e.g., a month, a week, a day, an hour, a minute, etc. For example, the function of time may be formed by plotting the various values of the resting heart rate on the ordinate and the sequence of the corresponding second time periods on the abscissa.

In an embodiment, predicting 2300 may comprise determining 2402 an inflection point $t_2$ (not shown) based on a pattern of change in the resting heart rate, wherein the pattern of change in the resting heart rate is defined by the resting heart rate increasing over time until the inflection point $t_2$ and decreasing after the inflection point $t_2$. For example, at the inflection point $t_2$, the function of the resting heart rate may take a value that is greater than the majority of the values of the resting heart rate in the previous time interval. For example, the function of the resting heart rate may exceed a predetermined threshold value. In an example, at the inflection point $t_2$, the function of the resting heart rate may take a value that is smaller than the majority of the values in the previous time interval. For example, the function of the resting heart rate may fall below a predetermined threshold value.

**Fig. 1d** illustrates a method of determining 2402 the inflection point $t_2$ using a regression function.

In an embodiment, determining 2402 the inflection point $t_2$ may comprise estimating 2402a a regression function using the resting heart rate, and determining 2402b the inflection point $t_2$ corresponding to a maximum of the regression function. A regression function may be a linear regression function. In one example, the values of the resting heart rate timely before the inflection point $t_2$ may be part of a first regression function indicating increasing values of the resting heart rate, and the values of the resting heart rate timely after the inflection point $t_2$ may be part of second regression function indicating decreasing values of the resting heart rate. For example, two (or more) regression functions may be combined to form a composite regression function. For example, a composite regression function may be continuous. For example, a continuous regression function may be non-differentiable at the inflection point $t_2$. For example, the regression function may comprise a polynomial regression function such as a quadratic regression function, or may comprise a multiple linear regression function.

Furthermore, an abscissa value, e.g., the inflection point $t_2$, may be determined at which the regression function reaches a maximum. A maximum may comprise a maximum in a certain interval. A maximum may be a global or a local maximum. In a preferred embodiment, the maximum is a global or absolute maximum of the regression function within the third time period $T_3$. For example, determining 2401 an inflection point $t_2$ may be performed sometime after inflection point $t_2$ is actually reached to verify that an absolute maximum occurred at the inflection point $t_2$. For example, the determining 2401 may be performed 3 weeks after the actual occurrence of the inflection point $t_2$ to confirm that the value of the resting heart rate has reached a global maximum at the inflection point $t_2$ and the decreasing trend of the resting heart rate continues after the inflection point $t_2$. Or, in other words, at any point in time (within the third time period $T_3$) the last one or more (for example 3 to 6) weeks may be examined for a global maximum of the heart rate or the inflection point in the function of the resting heart rate.

In an embodiment, predicting 2300 the date of delivery may comprise both determining 2401 an inflection point $t_1$ based on a pattern of change in the heart rate variability and determining 2402 an inflection point $t_2$ based on a pattern of change in the heart rate. In an example, the first time period of the heart rate variability function may be the same as the first time period of the resting heart rate function. In an example, the first time period of the heart rate variability function may be a different time period as the first time period of the resting heart rate function. In an example, the second time period of the heart rate variability function may be the same as the second time period of the resting heart rate function. In an example, the second time period of the heart rate variability function may be a different time period as the second time period of the resting heart rate function.

In an embodiment, predicting 2300 is based on the inflection point $t_1$ based on the pattern of change in the heart rate variability and on the inflection point $t_2$ based on the pattern of change in the heart rate. In an example, the use of both methods may be advantageous to compare the inflection point $t_1$ and inflection point $t_2$ with each other. For example, using both the inflection point $t_1$ based on the pattern of change in the heart rate variability and the inflection point $t_2$ based on the pattern of change in the heart rate may reduce noise, such that a resulting inflection point may be determined resulting from combining the inflection point $t_1$ and the inflection point $t_2$ by checking for consensus between when the heart rate variability function and the heart rate function both inflect. In an example, it may be advantageous to compute an average value from the two separated inflection points $t_1$ and $t_2$. In an example, the inflection point $t_1$ and inflection point $t_2$ may be the same point of time.

In an embodiment, a time period between the inflection point $t_1$ and/or the inflection point $t_2$ and the date of delivery may be about or exact 7 weeks. For example,

the inflection point $t_1$ indicating a pattern of change in the course of the heart rate variability and/or the inflection point $t_2$ indicating a pattern of change in the resting heart rate may be an indication that the birth may take place in 7 weeks (or 49 days) starting from the inflection point $t_1$ or the inflection point $t_2$. This may be used to predict, for example, a premature birth if the birth date calculated, e.g., by the doctor is dated at a later time, e.g., the predicted date of delivery will take place week 35 wherein the birth date calculated by the doctor is week 40. For example, in a preterm birth, shown in **Fig. 6a,** the inflection point may be at an earlier gestational age, e.g., 28th week of gestation, than in a full-term birth, shown in **Fig. 6b.** In an example, if 7 weeks to the date of delivery are calculated starting from the inflection point, an earlier gestation age at the inflection point may result in a preterm birth. The prediction may be advantageous to minimize the risks of a premature birth. In an embodiment, predicting the date of delivery, in particular for predicting a preterm birth, using heart rate data may be the indication for medical examinations such as testing for the presence of fetal fibronectin to verify a possible preterm birth by a doctor.

In an embodiment, the method 2000 may comprise receiving 2500 user-specific data via the user interface, wherein the user-specific data may comprise one or more of the first day of the last menstruation, a length of the menstrual cycle of the user, the day of conception, a pre-calculated date of delivery, or a combination thereof, wherein predicting 2300 the date of delivery is further based on the user-specific data. In an example, the date of delivery may be calculated theoretically based on the date of conception or a combination of the length of menstrual cycle and the first day of the last menstruation, on the basis that a pregnancy normally lasts 42 weeks. In another example, a pre-calculated date of delivery may be received via the user-interface. The pre-calculation of the date of delivery may be done by the doctor or the user may calculate the theoretical date of delivery using online tools. In an example, on the basis of the user-specific data a theoretical gestational age may be calculated. In an example, the current heart rate variability or the current (resting) heart rate may be compared to reference data, e.g., using a machine learning model to determine the current gestational age. For example, the machine learning model may be trained with data providing information about a typical heart rate variability or (resting) heart rate at a specific gestational age. In an example, a variety of machine learning models may be provided representing different reference groups depending on characteristic parameters, e.g., physical characteristics, age, medical history, race, or socioeconomic status. For example, the inflection point previously determined on the basis of the (measured) heart rate data can be matched using a calculated date of delivery. For example, a probability-based indication of the reliability of the prediction of the delivery date may be provided. In one example, the probability of a premature birth may be determined using the heart rate variability or the resting heart rate on the basis of the heart rate data and a date of delivery calculated based on the user-specific data. In an example the prediction of the date of delivery may be based on further data, such as change in sleep or exercise patterns causing the vital signs such as resting heart rate or heart rate variability to shift independently of the inflection point. The use of further data (e.g., motion data), which for example indicate diseases, may be advantageous. If the user gets sick at or around the inflection point, symptoms may suppress the vital signs and may delay the inflection, thereby creating the illusion of a later inflection point than might otherwise have occurred, presumably without actually shifting out the date of delivery.

In an embodiment, the method 2000 may further comprise presenting 2600 in a user interface the heart rate, the resting heart, and/or the heart rate variability as a function of time. For example, the time course of the heart rate, resting heart rate and/or heart rate variability may be displayed, for example, in the third time period $T_3$. In an example, the presentation of the heart rate, the resting heart rate, and/or heart rate variability may be in daily resolution as exemplary shown in **Fig. 5.** In an example, the presentation of the heart rate, the resting heart rate and/or heart rate variability may be in weekly resolution as exemplary shown in **Fig. 6a** or **Fig. 6b.** For example, a reference curve may be displayed that shows fluctuations in the heart rate, the resting heart rate or the heart rate variability that can normally occur during pregnancy. The user may thereby use the visualization to check whether her own heart rate, the resting heart rate, or heart rate variability shows a typical course compared to the reference curve and, for example, consult a doctor in the event of deviations. In an example, the reference curve may comprise a tolerance band indicating a range of typical values of a heart rate, resting heart rate or a heart rate variability. The reference curve may be predetermined by average values of a (training) data set corresponding to heart rates, resting heart rates, or heart rate variabilities of a plurality of other users. For example, by visualizing the course of the heart rate variability or the resting heart rate, a user may determine when an inflection point $t_1$ or an inflection point $t_2$ has been reached and may use the inflection point to determine when the birth will possibly take place according to the heart rate data. For example, if the calculated date of birth is known (for example based on the day of conception or the user-specific data), the user may determine whether there is a risk of premature birth using the visualization of the resting heart rate and/or heart rate variability.

In an embodiment, the method 2000 may further comprise presenting 2700 in a user interface a note to the user when the inflection point $t_1$ and/or the inflection point $t_2$ has been reached. For example, the note that the inflection point $t_1$ or the inflection point $t_2$ has been reached may be presented to the user a predetermined period of time after the actual inflection point $t_1$ or the inflection point $t_2$ when determining 2401 and/or determining 2402 has been completed, e.g. 3 weeks after the actual inflection point $t_1$ or the inflection point $t_2$, or at any other time when a reliable or statistically supported inference can be drawn concern a change in direction for the heart rate variability. For example, the note may include the information that the date of delivery will be in seven weeks starting from the inflection point $t_1$ or the inflection point $t_2$. In an example, the note may be a text message, an audible warning signal or a graphic presentation in the user interface.

In an embodiment, the method 2000 may further comprise presenting 2800 a warning to the user, when the inflection point $t_1$ and/or the inflection point $t_2$ indicates a preterm birth. In an example, the calculated date of delivery either calculated on the basis of the user-specific data or received via the user interface, may be used to be compared with the predicted date of delivery on the basis of the heart rate variability or on the basis of the resting heart rate as explained herein. If the date of the predicted date of delivery is earlier than the (pre-)calculated date of delivery, there may be a risk of premature birth. For example, premature birth can be defined as any birth between 20 and 37 weeks gestational age. For example, a full-term may be defined as any birth between 37 and 42 weeks gestational age. Post-term births may be defined as any birth taking place at 42 or more weeks gestational age. For example, the gestational age determined by methods explained herein may be used to define whether the date of delivery indicates a preterm birth.

In an embodiment, the method 2000 may further comprise presenting 2900 the remaining time until the date of delivery in a user interface. For example, a remaining time from the note issued to the user to the predicted date of delivery may be presented. In an example, starting from the inflection point $t_1$ or the inflection point $t_2$ there may be 7 weeks left until the date of delivery. In an example, determining 2401 or determining 2402 of the inflection point may be completed 3 weeks after the actual inflection point such that the note may be presented to the user 3 weeks after the actual inflection point indicating the remaining time until the date of delivery will be 4 weeks. In an example, the user interface may visualize the remaining time in the form of a timer constantly displaying the remaining time to the predicted date of delivery, e.g., in weekly or daily resolution.

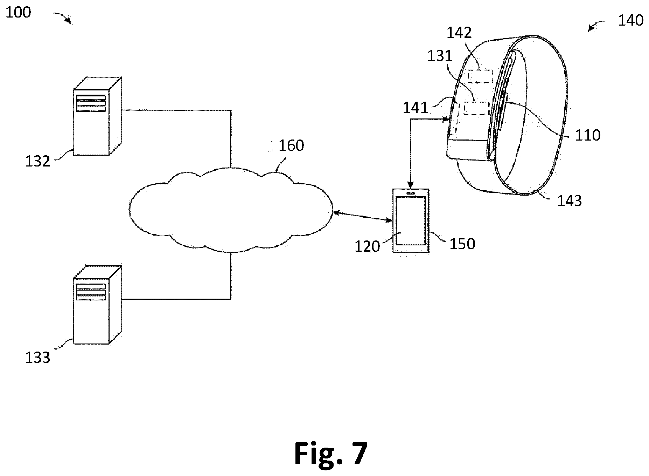

**Fig.** 7 illustrates a system 100 for predicting the date of delivery.

According to a second aspect, a system 100 for predicting a date of delivery comprises a sensor system 110 configured to acquire heart rate data for a pregnant user, a user interface 120 at least configured to present the predicted date of delivery, and a computational system configured to perform the computer-implemented method according to the first aspect. For example, the sensor system 110 may communicate with the computational system via a wired connection or a wireless connection, using techniques such as Bluetooth, Wi-Fi (Wireless-Fidelity), the mobile network (3G, 4G, 5G, ...), or near field communication. For example, also using Bluetooth, Wi-Fi (Wireless-Fidelity), the mobile network (3G, 4G, 5G, ...), or near field communication, the user interface 120 may communicate with the computational system and/or the sensor system 110 and may display, for example, the results of the predicting 2300. The user interface 120 may be part of or may comprise any computing device as described herein, including without limitation a smartphone, a desktop computer, a laptop computer, a network computer, a tablet, a mobile device, a portable digital assistant, a cellular phone, a portable media or entertainment device, and so on.

In an embodiment, the computational system may comprise a first processor 131 configured to acquire the heart rate data of a pregnant user. In an example, the computational system may comprise a first processor 131 configured to continuously acquire the heart rate data of a pregnant user. In this context, "continuously" will be understood to mean continuously as generally described herein. The system 100 may further comprise a wearable physiological monitoring device 140 comprising a communication interface 141 for coupling with a remote resource, wherein the sensor system 110 and the first processor 131 is integrated into the wearable physiological monitoring device 140. The wearable physiological monitoring device 140 may, in general, be any physiological monitoring device, such as any of the wearable monitors or other monitoring devices described herein, and may include a communication interface 141, one or more sensors of the sensor system 110, the first processor 131, a memory 142, and a wearable strap 143 for retaining the device in a desired location on a user. The communication interface 141 may be configured to couple one or more participants of the system 100 in a communicating relationship, e.g., with the remote resource using techniques such as Bluetooth, Wi-Fi (Wireless-Fidelity), the mobile network (3G, 4G, 5G, ...), or near field communication (NFC). In general, the one or more sensors of the sensor system 110 may include any of the sensors described herein, or any other sensors suitable for physiological monitoring. By way of example and not limitation, the one or more sensors of the sensor system 110 may include one or more of a light source, and optical sensor, an accelerometer, a gyroscope, a temperature sensor, a galvanic skin response sensor, an environmental sensor (e.g., for measuring ambient temperature, humidity, lighting, and the like), a geolocation sensor, a temporal sensor, an electrodermal activity sensor, and the like. In one aspect, the sensory system 110 includes optical sources such as LEDs along with photodetectors and accompanying processing for acquisition of heart rate data using photoplethysmography.

The first processor 131 and memory 142 may be any of the processors and memories described herein and suitable for deployment in the wearable physiological monitoring device 140. In one aspect, the memory 142 may store physiological data obtained by monitoring a user with the one or more sensors of the sensor system 110. The first processor 131 may be configured to obtain heart rate data from the user based on the data from the sensors of the sensor system 110. The first processor 131 may be further configured to assist in determining a heart rate variability or a resting heart rate and predicting the date of delivery as generally described herein. To this end, the first processor 131 may be configured to receive heart rate data from the sensor system 110 and to store the heart rate data in the memory 142. In an example, the first processor 131 may be configured to transmit at least some of the data from the memory 142 to the remote resource for determining a heart rate variability or a resting heart rate and/or predicting the date of delivery. In an example, the first processor 131 is configured to determine the heart rate variability (and/or resting heart rate) based on the heart rate data acquired by the physiological monitoring device 140, and is further configured to transmit the heart rate variability to the remote resource where further calculations such as an estimate of date of delivery can be performed. In another embodiment, the physiological monitoring device 140 may acquire raw signal data and transmit the raw signal data to a remote resource for calculation of heart rate, heart rate variability, and/or resting heart rate, as well as any other metrics of interest. In another example, the first processor 131 may be configured to determine the heart rate variability, predict the date of delivery and provide driving signals to a user interface for visualizing the result of the predicting the date of delivery.

In an embodiment, the computational system may further comprise a server 132 as the remote resource comprising a second processor at least configured by computer executable code to receive the heart rate data from the wearable physiological monitoring device 140, to determine 2200 a heart rate variability and/or a resting heart rate based on the received heart rate data, and to predict 2300 the date of delivery using the heart rate variability and/or the resting heart rate. In general, the server 132 may include data storage, the second processor, a communication interface, and/or other processing circuitry. The server 132 may process data from the wearable physiological monitoring device 140 and perform determining 2200 the heart rate variability and/or the resting heart rate and/or the predicting 2300 the date of delivery or any of the other analyses described herein, and may host a user interface 120 for remote access to this data, e.g., from a user device of one or more user devices 150. The server 132 may include a web server or similar front end that facilitates web-based access by the user devices 150 or the wearable physiological monitoring device 140 to the capabilities of the server 132 or other components of the system 100. In an example, the one or more user devices 150 may serve as a remote resource.

The other resources 133 may comprise any resources that can be usefully employed in the devices, systems, and methods as described herein. For example, these other resources 133 may include without limitation other data networks, sensors (e.g., audio or visual sensors), data mining tools, computational tools, data monitoring tools, algorithms, and so forth. The other resources 133 may also or instead include any other software or hardware resources that may be usefully employed in the networked applications as contemplated herein.

For example, the other resources 133 may comprise payment processing servers or platforms used to authorize payment for access, content, or option/feature purchases, or otherwise. In another aspect, the other resources 133 may comprise certificate servers or other security resources for third-party verification of identity, encryption or decryption of data, and so forth.

In another aspect, the other resources 133 may comprise a desktop computer or the like collocated (e.g., on the same local area network with, or directly coupled to through a serial or USB cable) with a user device of the one or more user devices 150, wearable strap 143, or server 132. In this case, the other resources 133 may provide supplemental functions for components of the system 100.

The other resources 133 may also or instead include one or more web servers that provide web-based access to and from any of the other participants in the system 100. While depicted as a separate network entity, it will be readily appreciated that the other resources 133 (e.g., a web server) may also or instead be logically and/or physically associated with one of the other devices described herein, and may, for example, include or provide a user interface 120 for web access to the server 132 or a database in a manner that permits user interaction through a data network 160, e.g., from the wearable physiological monitoring device 140 or the user device 150.

The data network 160 may be any of the data networks described herein. For example, the data network 160 may be any network(s) or internetwork(s) suitable for communicating data and information among participants in the system 100. This may include public networks such as the Internet, private networks, telecommunications networks such as the Public Switched Telephone Network or cellular networks using third generation (e.g., 3G or IMT-2000), fourth generation (e.g., LTE (E-UTRA) or WiMAX-Advanced (IEEE 802.16m)), fifth generation (e.g., 5G), and/or other technologies, as well as any of a variety of corporate area or local area networks and other switches, routers, hubs, gateways, and the like that might be used to carry data among participants in the system 100. This may also include local or short-range communications networks or communication protocols, e.g., Wi-Fi (Wireless-Fidelity), suitable, e.g., for coupling the wearable physiological monitoring device 140 to the user device 150, or otherwise communicating with local resources.

In an example, the one or more user devices 150 may work together with the wearable physiological monitoring device 140, e.g., to provide a display or the user interface 120 for user data and analysis, and/or to provide a communications bridge from the communication interface 141 of the wearable physiological monitoring device 140 to a data network 160 and the server 132. For example, the wearable physiological monitoring device 140 may communicate locally with a user device of the one or more user devices 150, such as a smartphone of a user, via short-range communications, e.g., Bluetooth, Wi-Fi (Wireless-Fidelity), near field communication (NFC), or the like, e.g., for the exchange of data between the wearable physiological monitoring device 140 and the user device 150, and the user device 150 may communicate with the server 132 via the data network 160. Computationally intensive processing, such as determining 2200 the heart rate variability and/or the resting heart rate and/or predicting 2300 the date of delivery, may be performed on the server 132, which may have greater memory capabilities and processing power than the wearable physiological monitoring device 140 that acquires the data.

The user device 150 may include any computing device as described herein, including without limitation a smartphone, a desktop computer, a laptop computer, a network computer, a tablet, a mobile device, a portable digital assistant, a cellular phone, a portable media or entertainment device, and so on. The user interface 120 may be part of the user device 150 and may provide access to data and analysis by a user, and/or to control operation of the wearable physiological monitoring device 140. The user interface 120 may be maintained by a locally-executing application on the user device 150, or the user interface 120 may be remotely served and presented on the user device 150, e.g., from the server 132 or from one or more other resources 133. It will be understood that two or more local devices (in addition to the physiological monitoring device 140) may also or instead be used. For example, one local device (e.g., a smart phone) may be used to receive data from the monitor and forward it to a remote server for processing, while another device (e.g., a laptop) may be used as a user interface for the user to investigate performance metrics, stress/strain history, delivery date estimates (and changes therein), and so forth.

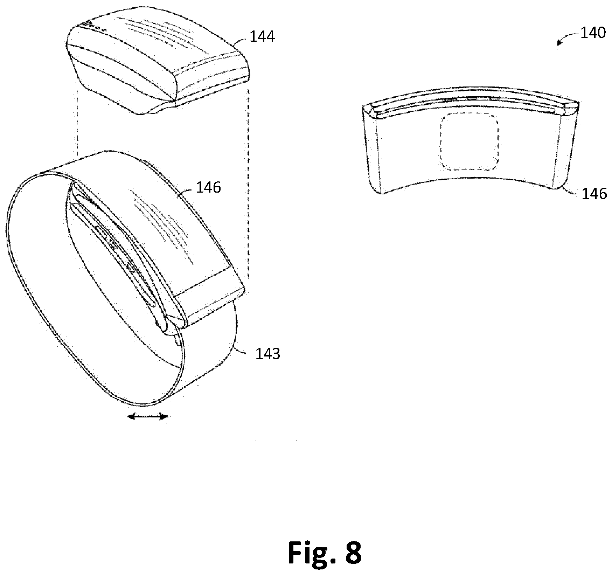

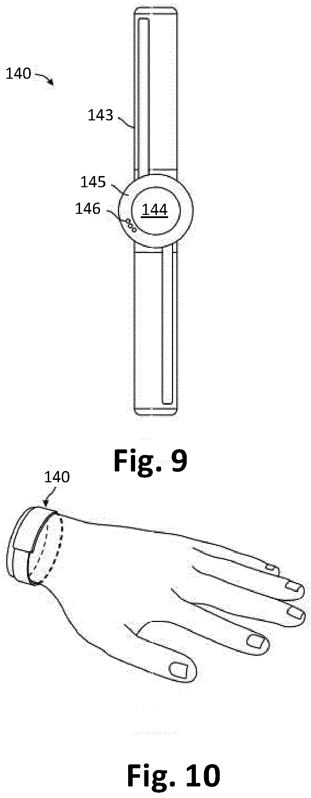

Fig. 8 illustrates front and back perspective views of a wearable physiological monitoring device 140 configured as a bracelet including one or more straps 143. Fig. 9 shows another exemplary embodiment of a bracelet according to aspects disclosed herein with an exemplary user interface 144. The bracelet may be sleek and lightweight, thereby making it appropriate for continuous wear. The bracelet may or may not include a display screen, e.g., user interface 144 such as a light emitting diode (LED) display for displaying any desired data (e.g., instantaneous heart rate, resting heart rate, or heart rate variability).

As shown in the non-limiting embodiment in **Fig. 8,** the strap 143 of the bracelet may have a wider side and a narrower side. In one embodiment, a user may simply insert the narrower side into the thicker side and squeeze the two together until the strap 143 is tight around the wrist, as shown in **Fig 10.** To remove the strap 143, a user may push the strap 143 further inwards, which unlocks the strap 143 and allows it to be released from the wrist. In other embodiments, various other fastening means may be provided. For example, the fastening mechanism may include, without limitation, a clasp, clamp, clip, dock, friction fit, hook and loop, latch, lock, pin, screw, slider, snap, button, spring, yoke, and so on.

In some embodiments, the strap 143 of the bracelet may be a slim elastic band formed of any suitable elastic material, for example, rubber. Certain embodiments of the wearable physiological monitoring device 140 may be configured to have one size that fits all. Other embodiments may provide the ability to adjust for different wrist sizes. In one aspect, a combination of constant module strap material, a spring-loaded, floating optical system and a silicon-rubber finish may be used in order to achieve coupling while maintaining the strap's comfort for continuous use. Use of medical-grade materials to avoid skin irritations may be utilized.

As shown in **Fig. 8,** the wearable physiological monitoring device 140 (e.g., the bracelet) may include components configured to provide various functions such as data collection and streaming functions of the system. In embodiments, the wearable system may include a button underneath the wearable system. In embodiments, the button may be configured such that, when the wearable physiological monitoring device 140 is properly tightened to one's wrist, the button may press down and activate the system to begin storing information. In other embodiments, the button may be disposed and configured such that it may be pressed manually at the discretion of a user to begin storing information or otherwise to mark the start or end of an activity period. In embodiments, the button may be held to initiate a time stamp and held again to end a time stamp, which may be transmitted, directly or through a mobile communication device application, to a website as a time stamp.

Time stamp information may be used, for example, as a privacy setting to indicate periods of activity during which physiological data may not be shared with other users. In one aspect, the button may be tapped, double-tapped (or triple-tapped or more), or held down in order to perform different functions or display different information (e.g., display battery information, generate time stamps, etc.). Other implementations may include more or less buttons or other forms of interfaces. More general, a privacy switch such as any of the user inputs or controls described herein may be operated to control restrictions on sharing, distribution, or use of heart rate or other continuously monitored physiological data. For example, the privacy switch may include a toggle switch to switch between a private setting where data is either not gathered at all or where data is stored locally for a user, and between a public, shared, or other non-private setting where data is communicated over a network and/or to a shared data repository. The privacy switch may also support numerous levels of privacy, e.g., using a hierarchical, role-based, and/or identity-based arrangement of permitted users and/or uses. As another example, various levels of privacy may be available for the type and amount of data that is shared versus private. In general, the privacy switch may be a physical switch on the wearable physiological monitoring device 140, or a logical switch or the like maintained on a computer or other local or mobile computing device of the user, or on a website or other network-accessible resource where the user can select and otherwise control privacy settings for monitored physiological data.

In embodiments, the wearable physiological monitoring device 140 may be waterproof so that users never need to remove it, thereby allowing for continuous wear.

**Fig. 10** illustrates placement of a wearable physiological monitoring device 140 on a user's wrist.

The wearable physiological monitoring device 140 (or "wearable monitor 140") may include a heart rate monitor (e.g., as part of the sensor system 110). In one example, the heart rate may be detected from the radial artery, in the exemplary positioning shown in **Fig. 10.** See, Certified Nursing Association, "Regular monitoring of your patient's radial pulse can help you detect changes in their condition and assist in providing potentially life-saving care." See, http://cnatraininghelp.com/cna-skills/counting-and-recording-a-radial-pulse. In another aspect, the wearable monitor 140 may be positioned anywhere on the body suitable for optical or other detection of cardiac activity based on sensing underlying vasculature. Thus, the wearable physiological monitoring device 140 may include a pulse sensor. In one embodiment, the wearable physiological monitoring device 140 may be configured such that, when a user wears it around their wrist and tightens it, the sensor portion (sensor system 110) of the wearable physiological monitoring device 140 is secured over the user's radial artery or other blood vessel(s). Secure connection and placement of the pulse sensor over the radial artery or other vasculature can facilitate sensing of heart rate and pulse. It will be understood that this configuration is provided by way of example only, and that other sensors, sensor positions, and monitoring techniques may also or instead be employed without departing from the scope of this disclosure.

In embodiments, the pulse or heart rate may be taken using an optical sensor (e.g., as part of the sensor system 1100) coupled with one or more light emitting diodes (LEDs), all directly in contact with the user's wrist. The LEDs are provided in a suitable position from which light can be emitted into the user's skin. In one example, the LEDs mounted on a side or top surface of a circuit board in the system to prevent heat build-up on the LEDs and to prevent burns on the skin. The circuit board may be designed with the intent of dissipating heat, e.g., by including thick conductive layers, exposed copper, heatsink, or similar. In one aspect, the pulse repetition frequency is such that the amount of power thermally dissipated by the LED is negligible. Cleverly designed elastic wrist straps can ensure that the sensors are always in contact with the skin and that there is a minimal amount of outside light seeping into the sensors. In addition to the elastic wrist strap, the design of the strap may allow for continuous micro adjustments (no preset sizes) in order to achieve an optimal fit, and a floating sensor module. The sensor module may be free to move with the natural movements caused by flexion and extension of the wrist.

In some embodiments, the wearable system may be configured to record other physiological parameters including, but not limited to, skin temperature (using a thermometer), galvanic skin response (using a galvanic skin response sensor), motion (using one or more multi-axis accelerometers and/or gyroscope), and the like, and environmental or contextual parameters, e.g., ambient temperature, humidity, time of day, and the like. In an implementation, sensors in the sensor system 110 are used to provide at least one of continuous motion detection, environmental temperature sensing, electrodermal activity (EDA) sensing, galvanic skin response (GSR) sensing, and the like. In this manner, an implementation can identify the cause of a detected physiological event. Reflectance PhotoPlethysmoGraphy (RPPG) may be used for the detection of cardiac activity, which may provide for non-intrusive data collection, usability in wet, dusty, and otherwise harsh environments, and low power requirements.

In embodiments, the wearable physiological monitoring device 140 may further be configured such that a button underneath the system may be pressed against the user's wrist, thus triggering the system to begin one or more of collecting data, calculating metrics, and communicating the information to a network. In embodiments, the sensor used for, e.g., measuring heart rate or GSR or any combination of these, may be used to indicate whether the user is wearing the wearable physiological monitoring device 140 or not. In embodiments, power to the one or more LEDs may be cut off as soon as this situation is detected and reset once the user has put the wearable system back on their wrist.

The wearable physiological monitoring device 140 may include one, two, or more sources of battery life, e.g., two or more batteries. In embodiments, it may have a battery that can slip in and out of the head of the wearable physiological monitoring device 140 and can be recharged using an included accessory. Additionally, the wearable physiological monitoring device 140 may have a built-in battery that is less powerful. When the more powerful battery is being charged, the user does not need to remove the wearable physiological monitoring device 140 and can still record data (during sleep, for example).

In embodiments, an application associated with data from an exemplary wearable physiological monitoring device 140 (e.g., a mobile communication device application) may include a user input component for receiving user-specific data.

In exemplary embodiments, the wearable physiological monitoring device 140 is enabled to automatically detect when the user is asleep, awake but at rest and exercising based on physiological data collected by the system.

**Fig. 9** schematically illustrates an exemplary embodiment of the wearable physiological monitoring device.

As shown in **Fig. 9,** a rotatable wheel 145 may be provided at the substantial center (or elsewhere) of the wearable physiological monitoring device 140 as a user input to control whether the system is displaying the heart rate or the heart rate variability. For example, when the wheel is turned to the right however, the system continuously shows heart rate or the heart rate variability and turns off the heart rate display when the wheel is turned to the right again. Turning the wheel to the left and holding it there forces data transmission to a mobile phone, external computer, or the Internet. In other embodiments, the wheel 145 may be absent in the wearable physiological monitoring device 140. In embodiments, the functionality of a rotatable wheel 145 described herein may be provided in an application of a mobile communication device (e.g., the user device 150) that is associated with physiological data collected from the wearable physiological monitoring device 140.

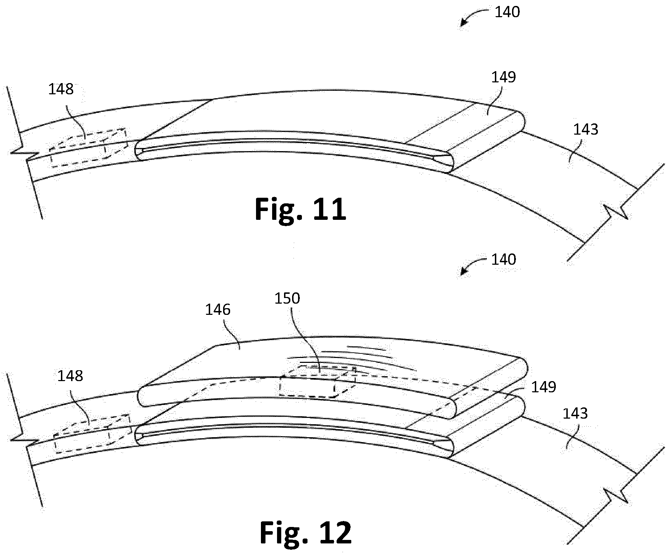

In embodiments, a physiological monitoring device 140 may be configured in a modular design to enable continuous operation of the system in monitoring physiological information of a user wearing the device. The module design may include a strap and a separate modular head portion or housing that is removably couplable to the strap. **Fig. 11** illustrates a side view of an exemplary physiological monitoring device 140 including a strap 143 that is not coupled to a modular head portion or housing 146. **Fig. 12** illustrates a side view of the wearable physiological monitoring device 140 in which the modular head portion 146 is removably coupled to the strap 143.

In the non-limiting illustrative module design, the strap 143 of a physiological monitoring device 140 may be provided with a set of components that enables continuous monitoring of at least a heart rate of the user so that it is independent and fully self-sufficient in continuously monitoring the heart rate without requiring the modular head portion 146. In one embodiment, the strap includes a plurality of light emitters for emitting light toward the user's skin, a plurality of light detectors for receiving light reflected from the user's skin, an electronic circuit board comprising a plurality of electronic components configured for analyzing data corresponding to the reflected light to automatically and continually determine a heart rate of the user, and a first set of one or more batteries for supplying electrical power to the light emitters, the light detectors and the electronic circuit board. In embodiments, the strap may also detect one or more other physiological characteristics of the user including, but not limited to, temperature, galvanic skin response, and the like. The strap may include one or more slots for permanently or removably coupling batteries 148 to the strap 143.

The strap 143 may include an attachment mechanism 149, e.g., a press-fit mechanism, for coupling the modular head portion 146 to the strap 143. The modular head portion 146 may be coupled to the strap 143 at any desired time by the user to impart additional functionality to the physiological monitoring device 140. In one embodiment, the modular head portion 146 may comprise a second set of one or more batteries 150 chargeable by an external power source so that the second set of batteries can be used to charge or recharge the first set of batteries 148 in the strap 143. The combination of the first and second sets of batteries enables the user to continuously monitor his/her physiological information without having to remove the strap for recharging. In embodiments, the module head portion may include one or more additional components including, but not limited to, an interface including visual display device configured to render a user interface for displaying physiological information of the user, a GPS sensor, an electronic circuit board (e.g., to process GPS signals), and the like.

Certain exemplary physiological monitoring devices may be configured to be coupled to any desired part of a user's body so that the system may be moved from one portion of the body (e.g., wrist) to another portion of the body (e.g., ankle) without affecting its function and operation. An exemplary physiological monitoring device 140 may include an electronic circuit board comprising a plurality of electronic components configured for analyzing data corresponding to the reflected light to automatically and continually determine a heart rate of the user. The electronic circuit board implements a processing module configured to detect an identity of a portion of the user's body, for example, an appendage like wrist, ankle, to which the strap is coupled based on one or more signals associated with the heart rate of the user, and, based on the identity of the appendage, adjust data analysis of the reflected light to determine the heart rate of the user.

In one embodiment, the identity of the portion of the user's body to which the physiological monitoring device is attached may be determined based on one or more parameters including, but not limited to, absorbance level of light as returned from the user's skin, reflectance level of light as returned from the user's skin, motion sensor data (e.g., accelerometer and/or gyroscope), altitude of the physiological monitoring device, and the like.

In embodiments, the processing module is configured to determine that the wearable physiological monitoring device 140 is taken off from the user's body. In one example, the processing module may determine that the wearable physiological monitoring device 140 has been taken off if data from the galvanic skin response sensor indicates data atypical of a user's skin. If the wearable physiological monitoring device is determined to be taken off from the user's body, the processing module is configured to deactivate the light emitters and the light detectors and cease monitoring of the heart rate of the user to conserve power.

Exemplary devices may comprise a processing module configured to filter the raw photoplethysmography data received from the light detectors to minimize contributions due to motion, and subsequently process the filtered data to detect peaks in the data that correspond with heart beats of a user. The overall algorithm for detecting heart beats takes as input the analog signals from optical sensors (mV) and accelerometer, and outputs an implied beats per minute (heart rate) of the signal accurate within a few beats per minute as that determined by an electrocardiography machine even during motion.

In one aspect, multiple LEDs with different wavelengths may reacting to movement in different ways, allowing for signal recovery under varying conditions with standard signal processing techniques. Accelerometer information can also be used, e.g., to compensate for coarse movement signal corruption. In order to increase the range of movements that the algorithm can successfully filter out, an aspect utilizes techniques that augment the algorithm already in place. For example, filtering may be applied to spurious data such as violent movements of the arm during very short periods of time, such as boxing as exercising, in order to improve the quality of acquired heart rate data. By selective sampling and interpolating over these impulses, an aspect can account for more extreme cases of motion. Additionally, an investigation into different LED wavelengths, intensities, and configurations can allow the systems described herein to extract a signal across a wide spectrum of skin types and wrist sizes. In other words, motion filtering algorithms and signal processing techniques may assist in mitigating the risk caused by movement.

In an embodiment, the user interface 120 may be configured to receive user-specific data from the user and to provide the user-specific data to the wearable physiological monitoring device 140 and/or the server 132. Using a software application visualized in the user interface 120, the user may be able to input user-specific data such as the first day of the last menstruation, a length of the menstrual cycle of the user, the day of conception, or a combination thereof. In an example, the data may be transmitted to the server to be used for predicting the date of delivery or may be used locally on the user device 150 to perform the predicting of the date of delivery.

In an embodiment, the computational system may comprise a memory configured to store at least the heart rate data, the user-specific data, the resting heart rate, and/or the calculated heart rate variability. The memory of the computational system may comprise the memory 142 integrated into the wearable physiological monitoring device 140 and/or may comprise memory of the server 132 and/or the other resources 133. In an example, the memory 142 of the wearable physiological monitoring device 140 may be configured to store at least the heart rate data received from the sensor system 110 and may be configured to transmit the heart rate data to the user device 150 which may comprise a memory for at least buffering the heart rate data. In an example, the user device 150 may be configured to transmit the data to the server where it may be stored in a server memory for further analyzing such as determining the heart rate variability and/or the resting heart rate and/or predicting the date of delivery.

According to a third aspect, a computer program product for predicting a date of delivery comprises a non-transitory computer-executable code, when executed on one or more computing devices, performs the steps of the method in accordance with the first aspect. The code may be performed, at least partially, on the server 132/other resources 133, the user device 150, and/or the wearable physiological monitoring device 140. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software.

According to a fourth aspect, a computer-readable medium is configured to store the computer program product in accordance with the third aspect. The computer program product may be stored in a non-transitory fashion in a computer memory, which may be a memory from which the program executes (such as random access memory associated with a processor), or a storage device such as a disk drive, flash memory or any other optical, electromagnetic, magnetic, infrared, or other device or combination of devices. In another aspect, any of the systems and methods described above may be embodied in any suitable transmission or propagation medium carrying computer-executable code and/or any inputs or outputs from same. In an example, the server 132, the other resources 133, the wearable physiological monitoring device 140, and/or the user device 150 may comprise memories configured to store the computer program product and may be configured to perform at least partial steps from the method in accordance with the first aspect. In another aspect, means for performing the steps associated with the processes described above may include any of the hardware and/or software described above. All such permutations and combinations are intended to fall within the scope of the present disclosure.

It will be understood that one or more of the steps related to any of the techniques for determining the heart rate variability and/or the resting heart rate and predicting the date of delivery as described herein, or sub-steps, calculations, functions, and the like related thereto, can be performed locally, remotely, or some combination of these. Thus, the one or more of the steps of **Fig. 1** may be performed locally on a wearable device, remotely on a server or other remote resource, on an intermediate device such as a local computer used by the user to access the remote resource, or any combination of these. For example, using the example system 100 of **Fig. 7,** one or more steps of a technique for predicting the date of delivery may, wholly or partially, be performed locally on one or more of the wearable physiological monitoring device 140, the user device 150, such as by using a trained machine learning model to compare the heart rate variability determined based on the measured heart rate data with the model or to determine the inflection point $t_1$, and then pruning or otherwise optimizing the machine learning model for deployment on the wearable physiological monitoring device 140. Also, or instead, one or more steps of a technique for predicting a date of delivery may, wholly or partially, be performed remotely on one or more of the server 132 and the other resource(s) 133. Thus, for example, when the wearable physiological monitoring device 140 is positioned near a smartphone of the user during sleep, heart rate data may be continuously or periodically transmitted to the server 132, which may use the received data to determine the heart rate variability, to determine the inflection point $t_1$, and to predict the date of delivery. Other combinations are also possible. For example, certain movement activity locally detected on a wearable device may be used to request an update to heart rate data and the like if/when necessary.

In certain aspects, only data necessary for determining 2200 the heart rate variability and/or the resting heart rate and/or predicting 2300 the date of delivery be transmitted from a local device (e.g., wearable physiological monitoring device 140 (or "wearable monitor 140") and/or the user device 150) over the data network 160 to one or more remote devices (e.g., the server 132 and/or other resource 133), or the most relevant or heavily weighted data may be preferentially transmitted before other available heart rate data. For example, this may include transmitting movement data before beginning transmission of the earliest available heart rate data from the device. In other aspects, more comprehensive data may be transmitted from the local device to the remote device for analysis thereof, but the remote device may first perform determining a heart rate variability and/or a resting heart rate before other, more computationally expensive analyses of the data are performed.

According to the foregoing disclosure, there are described herein a number of additional methods, systems and computer program products for predicting a delivery date. For example, in one aspect, a computer program product includes non-transitory computer readable code that, when executing on one or more computing devices, causes the computing devices to perform the steps of: acquiring data from a wearable monitor worn by a user during a pregnancy of the user, the data including heart rate data; calculating a history of heart rate variability for the user during the pregnancy by: identifying a number of periods of sleep for the user during the pregnancy based on the data from the wearable monitor, each of the number of periods of sleep associated with a calendar day; during each of the number of periods of sleep for the user, calculating a plurality of heart rate variability measurements over a plurality of intervals; for each of the number of periods of sleep for the user, aggregating the plurality of heart rate measurements into a heart rate variability; aggregating the heart rate variability into a plurality of weekly multi-day medians for heart rate variability for each of a plurality of consecutive weeks; identifying an inflection point in the history of heart rate variability at a day of a week where the weekly multi-day medians for heart rate variability change from a timewise decreasing value to a timewise increasing value; determining a predicted delivery date for an infant of the user at a predetermined number of days after the inflection point; and transmitting a notification to a user device of the user identifying the predicted delivery date.

Each of the plurality of intervals may be between four and six minutes. Each of the plurality of intervals may be separated by at least fifteen seconds. Aggregating the plurality of heart rate variability measurements into the heart rate variability may include calculating a weighted sum of the plurality of heart rate variability measurements. The weighted sum may weight the plurality of heart rate variability measurements more heavily toward an end of each sleep period. The weighted sum may weight the plurality of heart rate variability measurements according to a likelihood of occurring during a period of slow wave sleep. The wearable monitor may be a wearable photoplethysmography device.

In another aspect a method disclosed herein includes acquiring heart rate data from a wearable monitor worn by a user during a pregnancy of the user; calculating a history of a heart rate metric for the user during the pregnancy based on the heart rate data; and determining a predicted delivery date for the pregnancy based on a trend in the history of the heart rate metric for the user.

Determining the predicted delivery date may include identifying an inflection point in the history of the heart rate metric and calculating the predicted delivery date to occur a predetermined number of days after the inflection point. The history of the heart rate metric includes a history of heart rate variability for the user during the pregnancy. Each of a number of heart rate variability values in the history of heart rate variability may be calculated based on an aggregation of individual heart rate variability measurements acquired during a period of sleep by the user. Each of a number of heart rate variability values in the history of heart rate variability may be calculated as a weighted sum of the individual heart rate variability measurements. The weighted sum may weight heart rate variability measurements more heavily toward an end of the period of sleep. The weighted sum may weight heart rate variability measurements more heavily according to a likelihood of occurring during a period of slow wave sleep. The history of the heart rate metric may include a history of resting heart rate measurements for the user during the pregnancy. Calculating the history of the heart rate metric may include calculating a weekly sequence of seven day moving means for the heart rate metric based on the heart rate data. The heart rate metric may include at least one of a heart rate variability for the user and a resting heart rate for the user. Determining the predicted delivery date includes locating an inflection point in the heart rate metric from a first timewise decreasing value to a second timewise increasing value. Determining the predicted delivery date may include calculating the predicted delivery date at a predetermined number of days after the inflection point.

In another aspect, a system disclosed herein includes a wearable monitor, the wearable monitor configured to acquire physiological data from a user; and a server coupled in a communicating relationship with the wearable monitor, the server configured to calculate a predicted delivery date for the user by performing the steps of: acquiring heart rate data from the wearable monitor while worn by the user during a pregnancy, calculating a history of a heart rate metric for the user during the pregnancy based on the heart rate data, and calculating the predicted delivery date for the pregnancy at a predetermined number of days after an inflection point in the history of the heart rate metric from a timewise decreasing value in the heart rate metric to a timewise increasing value in the heart rate metric.

The system may include a user device, such as any of those described herein, configured to receive the physiological data (including the heart rate data) from the wearable monitor and forward the physiological data to the server. In this context, it will be understood that the physiological data received from the wearable monitor may include raw signal data from sensors of the wearable monitor, or processed data such as heart rate, resting heart rate, heart rate variability, and any other derived metric or the like that can be usefully calculated locally by the wearable monitor and forwarded to the server for use in delivery date predictions.

The above systems, devices, methods, processes, and the like may be realized in hardware, software, or any combination of these suitable for the control, data acquisition, and data processing described herein. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software.

Thus, in one aspect, each method described above, and combinations thereof may be embodied in computer executable code that, when executing on one or more computing devices, performs the steps thereof. In another aspect, the methods may be embodied in systems that perform the steps thereof, and may be distributed across devices in a number of ways, or all of the functionality may be integrated into a dedicated, standalone device or other hardware. The code may be stored in a non-transitory fashion in a computer memory, which may be a memory from which the program executes (such as random access memory associated with a processor), or a storage device such as a disk drive, flash memory or any other optical, electromagnetic, magnetic, infrared, or other device or combination of devices. In another aspect, any of the systems and methods described above may be embodied in any suitable transmission or propagation medium carrying computer-executable code and/or any inputs or outputs from same. In another aspect, means for performing the steps associated with the processes described above may include any of the hardware and/or software described above. All such permutations and combinations are intended to fall within the scope of the present disclosure.

The method steps of the implementations described herein are intended to include any suitable method of causing such method steps to be performed, consistent with the patentability of the following claims, unless a different meaning is expressly provided or otherwise clear from the context. So, for example, performing the step of X includes any suitable method for causing another party such as a remote user, a remote processing resource (e.g., a server or cloud computer) or a machine to perform the step of X. Similarly, performing steps X, Y, and Z may include any method of directing or controlling any combination of such other individuals or resources to perform steps X, Y, and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity and need not be located within a particular jurisdiction.

It will be appreciated that the methods and systems described above are set forth by way of example and not of limitation. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context.

**Claims**

1. A computer-implemented method (2000) for predicting a date of delivery for a pregnant user, comprising:

   acquiring (2100) heart rate data for the pregnant user from a wearable sensor system;
   determining (2200) a history of a heart rate variability based on the heart rate data;
   wherein the history of the heart rate variability is a function of time and comprises multiple values of a heart rate variability, wherein each value of the multiple values of the heart rate variability represents a mean or a median of the heart rate variability of a predetermined second time period; and
   predicting (2300) the date of delivery using the history of the heart rate variability, wherein the predicting (2300) comprises determining (2401) an inflection point $t_1$ based on a pattern of change in the history of the heart rate variability over a third time period $T_3$, wherein the pattern of change is defined by the multiple values of the heart rate variability in the history of the heart rate variability decreasing over time until the inflection point $t_1$ and increasing after the inflection point $t_1$, wherein the third time period $T_3$ indicates the gestational age at the time point of predicting (2300) the date of delivery.

2. The computer-implemented method (2000) according to claim 1, wherein the acquiring (2100) heart rate data is performed at night-time when the pregnant user is asleep.

3. The computer-implemented method (2000) according to claim 1 or 2, wherein the heart rate variability is a root-mean-square of time differences between two heartbeats in a first time period.

4. The computer-implemented method (2000) according to any one of the preceding claims, wherein the second time period corresponds to a week, a day, or a night.

5. The computer-implemented method (2000) according to any one of the preceding claims, wherein the third time period $T_3$ results from a sequence of second time periods.

6. The computer-implemented method (2000) according to any of the preceding claims, wherein the multiple values of the heart rate variability are determined over the course of the night, wherein optionally each of the multiple values is formed of a weighted average of values over different sleep phases.

7. The computer-implemented method (2000) according to claim 6, wherein the weighting favors periods of slow wave sleep, specific intervals within periods of slow wave sleep, periods of slow wave sleep having the highest quality, sleep periods closer to the end of a sleep period, and/or a combination thereof.

8. The computer-implemented method (2000) according to any one of the preceding claims, further comprising receiving (2500) user-specific data via a user interface, wherein the user-specific data comprises one or more of the first day of the last menstruation, a length of the menstrual cycle of the user, the day of conception, or a combination thereof, wherein predicting (2300) the date of delivery is further based on the user-specific data.

9. The computer-implemented method (2000) according to claim 7 or 8, further comprising presenting (2700) in a user interface a note to the user when the inflection point $t_1$ has been reached.

10. The computer-implemented method (2000) according to any one of claims 7 to 9, further comprising presenting (2800) a warning to the user, when the inflection point $t_1$ indicates preterm birth.

11. A system for predicting a date of delivery, comprising:

    a sensor system (110) configured to acquire heart rate data of a pregnant user,
    a user interface (120) at least configured to present the predicted date of delivery, and
    a computational system configured to perform the computer-implemented method according to the preceding claims.

12. The system according to claim 11, wherein the computational system comprises a first processor (131) configured to acquire the heart rate data for a pregnant user, the system further comprising a wearable physiological monitoring device (140) comprising a communication interface (141) for coupling with a remote resource, wherein the sensor system (110)

and the first processor (131) are integrated into the wearable physiological monitoring device (140).

13. The system according to claim 12, wherein the computational system comprises a server (132) as the remote resource comprising a second processor at least configured by computer executable code to receive the heart rate data from the wearable physiological monitoring device (140), to determine (2200) a history of a heart rate variability based on the received heart rate data, and to predict (2300) the date of delivery using the history of the heart rate variability

14. A computer program product for predicting a date of delivery, the computer program product comprising non-transitory computer-executable code, when executed on one or more computing devices, performs the steps of the method (2000) according to the claims 1 to 10.

15. A non-transitory computer-readable medium storing the computer program product according to claim 14.

**Patentansprüche**

1. Computerimplementiertes Verfahren (2000) zum Vorhersagen eines Geburtstermins für eine schwangere Benutzerin, umfassend:

Erfassen (2100) von Herzfrequenzdaten für die schwangere Benutzerin durch ein tragbares Sensorsystem;
Ermitteln (2200) einer Historie einer Herzfrequenzvariabilität basierend auf den Herzfrequenzdaten;
wobei die Historie der Herzfrequenzvariabilität eine Funktion der Zeit ist und mehrere Werte einer Herzfrequenzvariabilität umfasst, wobei jeder Wert der mehreren Werte der Herzfrequenzvariabilität einen Mittelwert oder einen Median der Herzfrequenzvariabilität eines vorbestimmten zweiten Zeitraums repräsentiert; und
Vorhersagen (2300) des Geburtstermins unter Verwendung der Historie der Herzfrequenzvariabilität, wobei das Vorhersagen (2300) ein Ermitteln (2401) eines Wendepunkts $t_1$ basierend auf einem Änderungsmuster in der Historie der Herzfrequenzvariabilität in einem dritten Zeitraum $T_3$ umfasst, wobei das Änderungsmuster durch die mehreren Werte der Herzfrequenzvariabilität in der Historie der Herzfrequenzvariabilität definiert wird, die im Laufe der Zeit bis zum Wendepunkt $t_1$ abnehmen und nach dem Wendepunkt $t_1$ zunehmen, wobei der dritte Zeitraum $T_3$ die Schwangerschaftsdauer zum Zeitpunkt des Vorhersagens (2300) des Geburtstermins anzeigt.

2. Computerimplementiertes Verfahren (2000) nach Anspruch 1, wobei das Erfassen (2100) von Herzfrequenzdaten während der Nacht durchgeführt wird, wenn die schwangere Benutzerin schläft.

3. Computerimplementiertes Verfahren (2000) nach Anspruch 1 oder 2, wobei die Herzfrequenzvariabilität ein quadratischer Mittelwert von Zeitdifferenzen zwischen zwei Herzschlägen in einem ersten Zeitraum ist.

4. Computerimplementiertes Verfahren (2000) nach einem der vorhergehenden Ansprüche, wobei der zweite Zeitraum einer Woche, einem Tag oder einer Nacht entspricht.

5. Computerimplementiertes Verfahren (2000) nach einem der vorhergehenden Ansprüche, wobei sich der dritte Zeitraum $T_3$ aus einer Sequenz von zweiten Zeiträumen ergibt.

6. Computerimplementiertes Verfahren (2000) nach einem der vorhergehenden Ansprüche, wobei die mehreren Werte der Herzfrequenzvariabilität im Verlauf der Nacht ermittelt werden, wobei jeder der mehreren Werte optional aus einem gewichteten Mittelwert von Werten während verschiedenen Schlafphasen gebildet wird.

7. Computerimplementiertes Verfahren (2000) nach Anspruch 6, wobei die Gewichtung Zeiträume des langsamwelligen Schlafs spezifische Intervalle innerhalb von Zeiträumen des langsamwelligen Schlafs, Zeiträume des langsamwelligen Schlafs, welche die höchste Qualität aufweisen, Schlafzeiträume näher am Ende eines Schlafzeitraums und/oder eine Kombination davon bevorzugt.

8. Computerimplementiertes Verfahren (2000) nach einem der vorhergehenden Ansprüche, das ferner ein Empfangen (2500) von benutzerspezifischen Daten über eine Benutzerschnittstelle umfasst, wobei die benutzerspezifischen Daten einen oder mehrere des ersten Tages der letzten Menstruation, einer Dauer des Menstruationszyklus der Benutzerin, des Tags der Empfängnis oder einer Kombination davon umfassen, wobei das Vorhersagen (2300) des Geburtstermins ferner auf den benutzerspezifischen Daten basiert ist.

9. Computerimplementiertes Verfahren (2000) nach Anspruch 7 oder 8, das ferner ein Darstellen (2700) einer Nachricht für die Benutzerin in einer Benutzeroberfläche umfasst, wenn der Wendepunkt $t_1$ erreicht wurde.

**10.** Computerimplementiertes Verfahren (2000) nach einem der Ansprüche 7 bis 9, das ferner ein Darstellen (2800) einer Warnung für die Benutzerin umfasst, wenn der Wendepunkt $t_1$ eine Frühgeburt anzeigt.

**11.** System zum Vorhersagen eines Geburtstermins, umfassend:

ein Sensorsystem (110), das konfiguriert ist, um Herzfrequenzdaten einer schwangeren Benutzerin zu erfassen,
eine Benutzeroberfläche (120), die mindestens konfiguriert ist, um den vorhergesagten Geburtstermin darzustellen, und
ein Computersystem, das konfiguriert ist, um das computerimplementierte Verfahren nach den vorhergehenden Ansprüchen durchzuführen.

**12.** System nach Anspruch 11, wobei das Computersystem einen ersten Prozessor (131) umfasst, der konfiguriert ist, um die Herzfrequenzdaten für eine schwangere Benutzerin zu erfassen, wobei das System ferner eine tragbare physiologische Überwachungsvorrichtung (140) umfasst, die eine Kommunikationsschnittstelle (141) zum Koppeln mit einer fernen Ressource umfasst, wobei das Sensorsystem (110) und der erste Prozessor (131) in die tragbare physiologische Überwachungsvorrichtung (140) integriert sind.

**13.** System nach Anspruch 12, wobei das Computersystem einen Server (132) als die ferne Ressource umfasst, die einen zweiten Prozessor umfasst, der zumindest durch einen in einem Computer ausführbaren Code konfiguriert ist, um die Herzfrequenzdaten von der tragbaren physiologischen Überwachungsvorrichtung (140) zu empfangen, um eine Historie einer Herzfrequenzvariabilität basierend auf den empfangenen Herzfrequenzdaten zu ermitteln (2200) und um den Geburtstermin unter Verwendung der Historie der Herzfrequenzvariabilität vorherzusagen (2300).

**14.** Computerprogrammprodukt zum Vorhersagen eines Geburtstermins, wobei das Computerprogrammprodukt einen nicht-flüchtigen in einem Computer ausführbaren Code umfasst, der, wenn er in einer oder mehreren Computervorrichtungen ausgeführt wird, die Schritte des Verfahrens (2000) nach einem der Ansprüche 1 bis 10 durchführt.

**15.** Nicht-flüchtiges computerlesbares Medium, in dem das Computerprogrammprodukt nach Anspruch 14 gespeichert ist.

**Revendications**

**1.** Procédé (2000) mis en œuvre par ordinateur, permettant de prédire une date d'accouchement d'une femme enceinte, comprenant les étapes consistant à :

acquérir (2100) des données de fréquence cardiaque de la femme enceinte depuis un système de capteur portable ;
déterminer (2200) un historique de variabilité de fréquence cardiaque en sur la base des données de fréquence cardiaque ;
dans lequel l'historique de la variabilité de fréquence cardiaque est une fonction du temps et comprend plusieurs valeurs d'une variabilité de fréquence cardiaque, chaque valeur parmi les multiples valeurs de la variabilité de fréquence cardiaque représentant une moyenne ou une médiane de la variabilité de fréquence cardiaque d'une deuxième période de temps prédéterminée ; et
prédire (2300) la date de l'accouchement à l'aide de l'historique de la variabilité de fréquence cardiaque, la prédiction (2300) consistant à déterminer (2401) un point d'inflexion $t_1$ sur la base d'un motif de variation de l'historique de la variabilité de fréquence cardiaque sur une troisième période temporelle $T_3$, le motif de variation étant défini par le fait que les valeurs multiples de la variabilité de fréquence cardiaque de l'historique de la variabilité de fréquence cardiaque diminuent au fil du temps jusqu'au point d'inflexion $t_1$, puis augmentent après le point d'inflexion $t_1$, la troisième période temporelle $T_3$ indiquant l'âge gestationnel au moment correspondant à la prédiction (2300) de la date d'accouchement.

**2.** Procédé mis en œuvre par ordinateur (2000) selon la revendication 1, dans lequel l'acquisition (2100) de données de fréquence cardiaque est effectuée la nuit, pendant que la femme enceinte est endormie.

**3.** Procédé mis en œuvre par ordinateur (2000) selon la revendication 1 ou 2, dans lequel la variabilité de fréquence cardiaque est une moyenne quadratique de différences temporelles entre deux battements cardiaques dans une première période de temps.

**4.** Procédé mis en œuvre par ordinateur (2000) selon l'une quelconque des revendications précédentes, dans lequel la deuxième période de temps correspond à une semaine, à un jour ou à une nuit.

**5.** Procédé mis en œuvre par ordinateur (2000) selon l'une quelconque des revendications précédentes, dans lequel la troisième période de temps $T_3$ résulte

d'une séquence de deuxièmes périodes de temps.

6. Procédé mis en œuvre par ordinateur (2000) selon l'une quelconque des revendications précédentes, dans lequel les valeurs multiples de la variabilité de la fréquence cardiaque sont déterminées au cours de la nuit, et dans lequel, facultativement, chacune des valeurs multiples est formée d'une moyenne de valeurs pondérée sur différentes phases de sommeil.

7. Procédé mis en œuvre par ordinateur (2000) selon la revendication 6, dans lequel la pondération utilise de préférence des périodes de sommeil lent profond, des intervalles spécifiques à l'intérieur de périodes de sommeil lent profond, des périodes de sommeil lent profond ayant la qualité la plus élevée, des périodes de sommeil plus proches de la fin d'une période de sommeil, et/ou une combinaison de celles-ci.

8. Procédé mis en œuvre par ordinateur (2000) selon l'une quelconque des revendications précédentes, comprenant en outre la réception (2500) de données spécifiques à l'utilisateur via une interface utilisateur, dans lequel les données spécifiques à l'utilisateur comprennent un ou plusieurs éléments parmi le premier jour de la dernière menstruation, une longueur du cycle menstruel de la femme, le jour de la conception, ou une combinaison de ceux-ci, la prédiction (2300) de la date d'accouchement étant en outre basée sur les données spécifiques à l'utilisateur.

9. Procédé mis en œuvre par ordinateur (2000) selon la revendication 7 ou 8, comprenant en outre la présentation (2700) dans une interface utilisateur d'une note à l'utilisateur au moment où le point d'inflexion $t_1$ a été atteint.

10. Procédé mis en œuvre par ordinateur (2000) selon l'une quelconque des revendications 7 à 9, comprenant en outre la présentation (2800) d'un avertissement à l'utilisateur, lorsque le point d'inflexion $t_1$ indique une naissance avant terme.

11. Système de prédiction d'une date d'accouchement, comprenant :

un système de capteurs (110) configuré pour acquérir des données de fréquence cardiaque d'un femme enceinte,
une interface utilisateur (120) au moins configurée pour présenter la date prévue pour l'accouchement, et
un système informatique configuré pour réaliser le procédé mis en œuvre par ordinateur selon les revendications précédentes.

12. Système selon la revendication 11, dans lequel le système informatique comprend un premier processeur (131) configuré pour acquérir les données de fréquence cardiaque d'une femme enceinte, le système comprenant en outre un dispositif de surveillance physiologique portable (140) comprenant une interface de communication (141) pour le couplage à une ressource distante, le système de détection (110) et le premier processeur (131) étant intégrés dans le dispositif de surveillance physiologique portable (140).

13. Système selon la revendication 12, dans lequel le système informatique comprend un serveur (132) servant de ressource distante et comprenant un deuxième processeur au moins configuré par un code exécutable par ordinateur pour recevoir les données de fréquence cardiaque provenant du dispositif de surveillance physiologique portable (140), afin de déterminer (2200) un historique d'une variabilité de fréquence cardiaque sur la base des données de fréquence cardiaque reçues, et de prédire (2300) la date de l'accouchement au moyen de l'historique de la variabilité de fréquence cardiaque

14. Produit-programme informatique permettant de prédire une date d'accouchement, le produit-programme informatique comprenant un code non transitoire exécutable par ordinateur qui, lorsqu'il est exécuté sur un ou plusieurs dispositifs informatiques, exécute les étapes du procédé (2000) selon les revendications 1 à 10.

15. Support non transitoire lisible par ordinateur stockant le produit-programme informatique selon la revendication 14.

**2000**

**2100**

**2200**

**2500**

**2300**

**2600**

**2700**

**2800**

**2900**

**Fig. 1a**

**2300**

**2401**

**2402**

**Fig. 1b**

**2401**

**2402**

**2401a**

**2401b**

**2402a**

**2402b**

**Fig. 1c**

**Fig. 1d**

2100

2101
Detect signal from sensor

2102
Determine condition of heart rate monitoring system

2103
Select mode based on condition

2104
Store continuous heart rate data

**Fig. 2**

2120

Detect sleep state of user

2130

Monitor heart rate of user

2140

Record heart rate as heart rate data

2150

Detect waking event

2200

**Determine heart rate variability and/or
resting heart rate in last phase of sleep**

2160

Evaluate quality of heart rate data using data
quality metric

# Fig. 3

Emit light using light emitters toward user's skin ⌐902

Detect light reflected from user's skin using light detectors ⌐904

Pre-process signals associated with reflected light ⌐906

Execute peak detection algorithm to detect peaks in pre-processed signals ⌐908

Determine an RR interval based on detected peaks ⌐910

Determine confidence level associated with RR interval ⌐912

⌐914 Confidence level > threshold ?

**Yes**　　　　　　　　　　　　　　　　**No**

Use detected peaks to determine instantaneous heart rate ⌐916

Determine heart rate variability ⌐918

Execute frequency analysis algorithm to determine instantaneous heart rate based on pre-processed signals associated with reflected light ⌐920

Determine heart rate variability ⌐922

# Fig. 4

# Fig. 5

## Fig. 6a

## Fig. 6b

**Fig. 7**

144

140

146

143

146

**Fig. 8**

140

143

145

146 144

**Fig. 9**

140

**Fig. 10**

**Fig. 11**

**Fig. 12**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63401406
- US 2022192585 A1, BILIC ALJOSA
- US 10264982 B

**Non-patent literature cited in the description**

- **ROWAN SHON P. et al.** Monitoring One Heart to Help Two: Heart Rate Variability and Resting Heart Rate using Wearable Technology in Active Women Across the Perinatal Period. *medRxiv*, 23 April 2022
- **SARHADDI FATEMEH et al.** Trends in Heart Rate and Heart Rate Variability During Pregnancy and the 3-Month Postpartum Period: Continuous Monitoring in a Free-living Context. *JMIR MHEALTH AND UHEALTH*, 03 June 2022, vol. 10 (6)